# EUROPEAN PATENT APPLICATION

(11) **EP 4 331 658 A1**
(43) Date of publication of application: **06.03.2024**
(21) Application number: 22836648.0
(22) Date of filing: 31.05.2022
(51) Int. Cl.: A61M 37/00, A61K 9/00, A61K 38/22, A61K 38/28, A61K 45/00, A61K 47/10, A61K 47/26, A61K 47/32, A61P 3/10

(54) **STRONGLY HYDROPHILIC MICRONEEDLE SUBSTRATE AND DRUG-LOADED MICRONEEDLE AND USE THEREOF IN TREATMENT OF DISEASES**

(30) Priority: 06.07.2021 CN 202110764097; 09.07.2021 CN 202110779410
(71) Applicant: Yin, Zhong, Gulou Nanjing, Jiangsu 210036 (CN)
(72) Inventor: Yin, Zhong, Gulou Nanjing, Jiangsu 210036 (CN)
(74) Representative: karo IP
(86) International application number: PCT/CN2022/096295
(87) International publication number: WO 2023/279893

(57) **Abstract**

A microneedle made of a strongly hydrophilic substrate. A composition of polyvinyl alcohol and a small molecule polyhydroxy compound is used as a substrate to prepare a microneedle having a strongly hydrophilic surface and a drug-loaded microneedle. The microneedle having a strongly hydrophilic surface can be obtained by means of the compatibility of a polyhydroxy polymer polyvinyl alcohol and a small molecule polyhydroxy compound. The microneedle can strongly adsorb a drug as long as the microneedle is in contact with the drug, the drug does not easily fall off, the process is simple, and mass production is facilitated. The drug-loading capacity is significantly increased, and the drug use efficiency is high. The preparation of a blank microneedle and the drug loading are conducted in stages and the drug loading stage is not conducted at a high temperature. Therefore, the microneedle can be effectively used in drug loading and drug release in the treatment of diseases, and is easy for mass production. By means of the microneedle substrate composition, the blank microneedle prepared therefrom and the use of the blank microneedle in drug loading, the inherent surface strongly hydrophilic polarity of the polyvinyl alcohol is maintained, such that the drug-loading capacity and the *in-vivo* penetration capacity of the coated drug loading are significantly increased.

## Description

### TECHNICAL FIELD

The present disclosure relates to a microneedle made of a strongly hydrophilic substrate, and more specifically relates to a microneedle having a strongly hydrophilic surface and a drug-loaded microneedle prepared by using a composition of polyvinyl alcohol and a small molecule polyhydroxy compound as a substrate, and use thereof in treatment of diseases.

### BACKGROUND

The drug microneedle has been developed rapidly in recent years as a drug-loading and release means, but the practical application of mass production is relatively slow. Although various related arts are different in the microneedle preparation, drug-loading method, and the like, the application is limited by the microneedle molding method and drug-releasing method.

The drug-loading method of the drug microneedle mainly includes two types: 1, mixed drug loading: uniformly mixing a drug and a microneedle substrate powder into a whole, and then processing a mixture containing a drug into a microneedle; and 2. Coated drug loading: firstly, separately processing a microneedle substrate material into a blank microneedle, and then loading a drug to a tip of the microneedle in a low-temperature environment. The two drug-loading methods each have advantages and disadvantages. The mixed drug-loading capacity is not large enough, and the coated drug-loading capacity is smaller and even many times smaller. Although the coated drug-loading capacity is smaller and the drug is easy to fall off, the loaded drug is positioned on the surface of the microneedle tip and directly contacts with human tissues, and the drug release is fast and relatively sufficient, the absorption is complete, and the drug is saved. The drug of the mixed drug loading is embedded in the whole microneedle body. Although the drug-loading capacity is higher than that of the coated drug loading, only the drug at the tip of the microneedle can enter human tissues. The drug-releasing process is blocked by the needle tip substrate, the drug release is slow, and more drugs not having an effect remain at a far end of the needle body. The coated drug is easy to fall off from the surface of the microneedle and also easy to be pressed after being extruded into a skin hole, and a part of the drug is pushed out of the skin. The easy falling off, small drug-loading capacity, and extrusion out of the skin of the coated drug loading are generally caused by insufficient adhesion (affinity) between the surface of the microneedle and the drug.

There are two general molding methods of the drug microneedle: 1. non-mold molding (such as a wire-drawing method and a 3D printing method); and 2. molding by a mold. There are three mold-molding methods: 1. hot injection method of mixed materials: the drug in the material is subjected to a relatively high temperature and thus the method cannot be used for most protein and polypeptide drugs; 2. cold injection method of concentrated solution of mixed materials: the method can be conducted at a low temperature, the quality of the drug is ensured, but the drying process consumes long time, and the amplification of mass production is limited; and 3. hot-pressing cold-coating method: firstly, a thermoplastic substrate is hot-pressed into a blank microneedle, then the drug is coated at a low temperature, the method is simple and convenient in process and easy for amplification of mass production, but the drug-loading capacity is very small, the drug is easy to fall off, and after the needle tip is pushed into the skin, a part of the drug can be extruded out of the skin.

In summary, the main technical problems of the drug microneedle are: the mixed drug-loading method has a low drug utilization rate and poor comprehensive benefit; the drug-loading capacity is small, especially the coated drug-loading capacity is smaller and the drug is easy to fall off; many methods are conducted at a high temperature and thus are not suitable for many drugs; and the process is relatively complicated, various conditions need to be precisely controlled, and the amplification of mass production is difficult.

### SUMMARY

The present disclosure provides a microneedle substrate composition which can be used for preparing a microneedle through a relatively simple technical process, a blank microneedle prepared by the substrate composition, and a drug contacted and loaded by the blank microneedle, such that the microneedle can be used in the form of a drug preparation for treating diseases.

The present disclosure adopts the following technical solutions:
A strongly hydrophilic microneedle substrate composition, including a small molecule polyhydroxy compound and polyvinyl alcohol, a hydroxyl ratio of the small molecule polyhydroxy compound to the polyvinyl alcohol being 0.1:1 to 1.3:1, the polymerization degree of the polyvinyl alcohol being 300-3,500, and the alcoholysis degree being 80% or more.

The polymerization degree of the polyvinyl alcohol is 300-2,600.

The alcoholysis degree of the polyvinyl alcohol is 88% or more.

The hydroxyl ratio of the small molecule polyhydroxy compound to the polyvinyl alcohol is 0.15:1 to 1:1.

The small molecule polyhydroxy compound is selected from one or more of glycerol, erythritol, threitol, xylitol, arabitol, ribitol, sorbitol, mannitol, galactitol, iditol, volemitol, ribose, xylose, and glucose.

The small molecule polyhydroxy compound is selected from one or more of glycerol, erythritol, xylitol, sorbitol, and mannitol.

A strongly hydrophilic microneedle, a microneedle substrate being the strongly hydrophilic microneedle substrate composition.

A strongly hydrophilic microneedle substrate, the strongly hydrophilic microneedle substrate composition being mixed into a blended powder, or the strongly hydrophilic microneedle substrate composition being mixed and hot-pressed into a molded material, or the strongly hydrophilic microneedle substrate composition being hot-mixed and melt-extruded by a blending machine into a molded material.

A method for preparing a strongly hydrophilic microneedle, including:
a) blending the strongly hydrophilic microneedle substrate composition at room temperature to form a blended powder; and
b) hot-pressing the blended powder into a molded material and hot-pressing the molded material into a mold to form a blank microneedle; or hot-mixing and melt-extruding the blended powder by a blending machine at a certain temperature into a molded material, and hot-pressing the molded material into a mold to form a blank microneedle.

The mold is a flat plate with one or more groups of micropore arrays, the micropore is a through hole, and the thickness of the flat plate is 0.2-3 mm; and the molded material is hot-pressed into the mold to form the blank microneedle, namely the blended powder or the molded material is added on the micropore array of the mold plate, and heated to form a melt flow material able to flow, a pressure is applied from an upper part of the material downwards, or vacuumizing is conducted from a lower part of the mold to reduce pressure, or a pressure is applied from the upper part and vacuumizing is conducted from the lower part at the same time so as to enable the melt flow material to enter the micropores of the mold.

The pressure is 28-32 kg/cm², and a temperature of the hot-pressing or hot-mixing is from a molding lower limit temperature of the substrate to a decomposition temperature of polyvinyl alcohol.

A drug-loaded microneedle, the drug-loaded microneedle being formed by contacting and adsorbing a solution containing a drug on a surface of a tip of the strongly hydrophilic microneedle and drying the microneedle.

The drug is a protein drug, a polypeptide drug or a small molecule drug.

The drug is one of insulin, exenatide, human growth hormone, and finasteride.

The solution containing a drug is an aqueous solution prepared when the drug is water-soluble, or a solution or suspension prepared by uniformly suspending the drug in water or dissolving/suspending the drug in a non-aqueous polar solvent when the drug is water-insoluble.

When the drug is water-soluble, the solution containing a drug consists of a drug, povidone or polyvinyl alcohol, and water, where the concentration of the povidone or the polyvinyl alcohol is 0.5%-15%; when the active drug is water-insoluble, the solution containing a drug consists of a drug, povidone or polyvinyl alcohol, and water and/or a non-aqueous polar solvent, where the concentration of the povidone or the polyvinyl alcohol is 4%-15%; and the non-aqueous polar solvent is one or a mixed solvent of methanol, ethanol, and isopropanol.

The contacting and adsorbing process includes: forming a groove with the depth of 0.1-0.7 mm in a flat plate, adding the solution containing a drug into the groove to a certain height, enabling a tip of the strongly hydrophilic microneedle to vertically face downwards, enabling the microneedle to stand and stay in the circular groove, taking out the microneedle, and drying the microneedle to obtain the drug-loaded microneedle.

A drug-loaded microneedle for combined administration, formed by integrally combining two or more microneedles each loading a different drug of the drug-loaded microneedle.

The drug-loaded microneedle for combined administration, formed by integrally combining an insulin drug-loaded microneedle and an exenatide drug-loaded microneedle, where the insulin drug-loaded microneedle and the exenatide drug-loaded microneedle are separately arranged.

An insulin drug-loaded microneedle, where in the drug-loaded microneedle, the drug is insulin.

An exenatide drug-loaded microneedle, where in the drug-loaded microneedle, the drug is exenatide.

Through a large number of theoretical and test researches, it is found that the microneedle having a strongly hydrophilic surface can be obtained by means of the compatibility of a polyhydroxy polymer polyvinyl alcohol and a small molecule polyhydroxy compound. The microneedle can strongly adsorb a drug as long as the microneedle is in contact with the drug, the drug does not easily fall off, the process is simple, and mass production is facilitated. The drug-loading capacity is significantly increased, and the drug use efficiency is high. The preparation of a blank microneedle and the drug loading are conducted in stages and the drug loading stage is not conducted at a high temperature. Therefore, the microneedle can be effectively used in drug loading and drug release in the treatment of diseases, and is easy for mass production. The present disclosure is finished.

By means of the microneedle substrate composition, the blank microneedle prepared therefrom and the use of the blank microneedle in drug loading, a microneedle administration solution is provided for treating diseases. The solution has the following remarkable characteristics and beneficial effects:
I. In the microneedle composition substrate of the present disclosure, a ratio of a small molecule polyhydroxy compound and polyvinyl alcohol is used to adjust physical properties, the thermoplasticity of the polyvinyl alcohol is improved, and a result that a thermoplastic temperature is greatly lower than a decomposition temperature is achieved.
II. The inherent surface strongly hydrophilic polarity of the polyvinyl alcohol is maintained, such that the drug-loading capacity and the *in-vivo* penetration capacity of the coated drug loading are significantly increased.
III. Since the drug loading is conducted by means of hydrophilic polar adsorption, the preparation of the blank microneedle and the drug loading can be conducted step by step, and the drug-loading process can be conducted at a low temperature zero or above, such that the quality of the drug is ensured, and the microneedle is suitable for administration of many drugs, particularly the drugs sensitive to temperature such as proteins and polypeptides.
IV. The drug is coated on the surface of a needle tip and directly contacts with the intradermal tissue, and thus takes effect fast, and has a high utilization rate and a cost advantage.
V. A needle body has the proper hardness, high administration precision, and mild skin feel.
VI. The microneedle substrate has the good toughness, meets the stress requirement of machining, and has a simple and clear process, simple and convenient operation, extremely short time consumption, and great process amplification potential.

These remarkable characteristics and beneficial effects are all based on the attribute characteristics of the microneedle composition involved in the present disclosure, namely: 1. biological safety; 2. strong surface adhesiveness; 3. good thermoplasticity; 4. specific affinity relationship with aqueous solution; 5. good cold shrinkage and toughness; and 6. natural degradability. Based on the attribute characteristics, the microneedle composition substrate involved in the present disclosure powerfully supports the medicinal prospect, the industrial mass production prospect, and the environmental protection requirements of the drug microneedle.

### BRIEF DESCRIPTION OF THE DRAWINGS

FIG. 1 is a schematic diagram of a dedicated microneedle preparation device;
FIG. 2 is a schematic diagram of a mold plate;
FIG. 3 is a schematic diagram of a process of preparing a microneedle after directly preparing a blended powder into a molded material;
FIG. 4 is a schematic diagram of a process of preparing a microneedle by hot-mixing and extruding a blended powder into a molded material by a machine;
FIG. 5 is a schematic diagram of a process of preparing a microneedle by preheating a device to a molding temperature and then placing a molded material for hot-pressing;
FIG. 6 is an image of a blank microneedle of the present disclosure under a high power magnifying glass (30x);
FIG. 7 is a schematic diagram of the blank microneedle of the present disclosure;
FIG. 8 is an image of a drug firmly adsorbed at a needle tip, after the blank microneedle of the present disclosure is loaded with a drug under a high power magnifying glass (30x);
FIG. 9 is a schematic diagram of a drug-loaded microneedle of the present disclosure;
FIG. 10 is a schematic diagram showing a state that a microneedle hangs a drug liquid to coat the tip of the microneedle;
FIG. 11 is a standard H-NMR of manicol;
FIG. 12 is a self-tested H-NMR of commercially available polyvinyl alcohol 1788;
FIG. 13 is an H-NMR of a polyvinyl alcohol 1788/manicol (1:0.6185 w/w) microneedle tablet;
FIG. 14 is an H-NMR of a majority of polyvinyl alcohol precipitated by cupric ions of a polyvinyl alcohol 1788/manicol (1:0.6185 w/w) microneedle tablet aqueous solution;
FIG. 15 is an H-NMR of a majority of a supernatant manicol after precipitation by cupric ions of a polyvinyl alcohol 1788/manicol (1:0.6185 w/w) microneedle tablet aqueous solution;
FIG. 16 is a self-tested H-NMR of commercially available polyvinyl alcohol 1799;
FIG. 17 is an H-NMR of a polyvinyl alcohol 1799/manicol (1:0.689 w/w) microneedle tablet;
FIG. 18 is an H-NMR of a majority of polyvinyl alcohol precipitated by cupric ions of a polyvinyl alcohol 1799/manicol (1:0.689 w/w) microneedle tablet aqueous solution;
FIG. 19 is an H-NMR of a majority of a supernatant manicol after precipitation by cupric ions of a polyvinyl alcohol 1799/manicol (1:0.689 w/w) microneedle tablet aqueous solution;
FIG. 20 is a schematic diagram of a combined microneedle;
FIG. 21 shows a drug effect of an insulin microneedle in a model pig of diabetes;
FIG. 22 shows a drug effect of an exenatide microneedle in a model pig of diabetes;
FIG. 23 is a table showing molding temperatures of prefabricated wafers and/or microneedles by different hot-extruded materials;
FIG. 24 is a table showing compositions of blended powders, i.e., molded materials for testing;
FIG. 25 is a table showing results of adhesion tests of substrates to spots formed by a sodium chloride/povidone solution;
FIG. 26 is a table showing results of amounts of test solutions adsorbed by microneedles;
FIG. 27 is a table showing the state of drug liquid drops hanging on the needle tip observed under a high power magnifying glass (30x);
FIG. 28 is a table showing the falling-off of the drug observed under a high power magnifying glass (30x) after a one-meter-high free fall;
FIG. 29 is a table showing results of the toughness and friability of microneedle substrates;
FIG. 30 is a table showing the amount of test solutions of water-soluble materials adsorbed by microneedles;
FIG. 31 is a table showing amounts of test solutions of water-insoluble materials adsorbed by microneedles;
FIG. 32 is a table showing drug-effect tests of insulin drug-loaded microneedles;
FIG. 33 is a table showing drug-effect tests of exenatide drug-loaded microneedles;
FIG. 34 is a table showing molded materials of wafers directly pressed by blended powder materials;
FIG. 35 is a table showing molded materials of wafers pressed by extruded particle materials;
FIG. 36 is a table showing integrity of bodies of microneedles with five different material ratios separated from the mold plate;
FIG. 37 is a table showing hot-dissolved removal of mannitol/XX99 polyvinyl alcohols of different polymerization degrees;
FIG. 38 is a table showing results of tests of form retention of material needle bodies with different alcoholysis degrees in skin holes.

### DETAILED DESCRIPTION

The present disclosure is described in detail in conjunction with the accompanying drawings and specific examples below.

Specific implementations of the present disclosure are described below in conjunction with the accompanying drawings, but do not limit the present disclosure.

Firstly, the present disclosure provides a strongly hydrophilic microneedle substrate composition, including polyvinyl alcohol and a small molecule polyhydroxy compound, a hydroxyl ratio of the small molecule polyhydroxy compound to the polyvinyl alcohol being 0.1:1 to 1.3:1, the polymerization degree of the polyvinyl alcohol being 300-3,500, and the alcoholysis degree being 80% or more.

The polymerization degree in the present disclosure refers to the total number of structural units in a polymer molecule, that is, the number of the structural units polymerized to form a polymer. Polyvinyl alcohol is generally obtained by polymerization of vinyl acetate and alcoholysis, where the vinyl acetate units are alcoholyzed to form vinyl alcohol units, where a portion thereof is alcoholyzed and a left portion thereof is non-alcoholyzed. Therefore, the extent of alcoholysis is expressed in terms of the alcoholysis degree, i.e., the alcoholysis degree is the percentage of the number of vinyl alcohol units in the polyvinyl alcohol molecule accounting for the total number of the units, and the remaining non-alcoholyzed units are vinyl acetate. The alcoholysis degree is closely related to physical properties of the polyvinyl alcohol, such as water solubility, hydrophilicity, softening temperature and mechanical strength. When the polyvinyl alcohol is matched with other substances, such as a polyhydroxy substance, hydrogen bonds can be formed between the two substances. The hydrogen bonds are different in a certain degree according to different mixing ratios, thereby affecting the physical properties. Therefore, the present inventors obtain a strongly hydrophilic microneedle substrate composition through intensive researches and tests.

The polymerization degree and the alcoholysis degree are represented by the four-digit number. The first two digits represent the polymerization degree and the last two digits represent the alcoholysis degree, for example, 3588 polyvinyl alcohol has the polymerization degree of 3,500 units and the alcoholysis degree of 88%. In the following description, unless otherwise specified, the four-digit number indicates the polyvinyl alcohol having the polymerization degree and the alcoholysis degree. Corresponding polyvinyl alcohols are all commercially available. When polyvinyl alcohols of special specifications are required for testing the performances of the present disclosure, the polyvinyl alcohols are used after synthesized and characterized according to the methods of the related art (reference: Qiu Tianrong, Control of Alcoholysis Degree for Polyvinyl Alcohol Production, Chemical Engineering & Equipment, 2009, Issue IX, P66-70).

The physical properties of the obtained substrate mainly depend on the polymerization degree and alcoholysis degree of the polyvinyl alcohol and the ratio of added small molecule polyhydroxy compound, and depend on the types of the small molecule polyhydroxy compound to a lesser extent. The influence degrees of the factors can be obtained from a test.

Preferably, the polyvinyl alcohol has the polymerization degree of 300-3,500 and the alcoholysis degree of 88%-100%. In accordance with practice, the alcoholysis degree of the fully alcoholyzed polyvinyl alcohol is denoted as XX99, not XX100.

Preferably, the polymerization degree of the polyvinyl alcohol is 300-2,600.

Preferably, the alcoholysis degree is 88% or more.

Preferably, the hydroxyl ratio is 0.15:1 to 1:1.

The small molecule polyhydroxy compound is selected from one or more of glycerol, erythritol, threitol, xylitol, arabitol, ribitol, sorbitol (sorbol), mannitol (manicol), galactitol, iditol, volemitol, ribose, xylose, and glucose.

Preferably, the small molecule polyhydroxy compound is selected from one or more of glycerol, erythritol, xylitol, sorbitol, and mannitol.

The polyvinyl alcohol molecules contain a large number of hydroxyl groups (the amount of the hydroxyl groups is characterized by the alcoholysis degree), and can form tight intramolecular or intermolecular hydrogen bonds, such that the melting temperature of the polyvinyl alcohol molecules is higher than the decomposition temperature, and thermoplasticity barely exists; after the small molecule polyhydroxy compound is mixed in the polyvinyl alcohol, the intramolecular or intermolecular hydrogen bonds of the polyvinyl alcohol molecules are partially loosened and weakened, and the thermoplasticity is increased; and after the small molecule polyhydroxy compound is heated and converted into liquid, the melt flow property of the material is improved, namely, after the small molecule polyhydroxy compound is added, the two functions are both beneficial to the thermoplastic molding of the mixed material. Therefore, the thermoplasticity of the composition can be controlled by adjusting the addition amount of the small molecule polyhydroxy compound.

The addition amount can be estimated from a molecular level: each hydroxyl group of the small molecule polyhydroxy compound can react with one hydroxyl group of polyvinyl alcohol, and the ratio relationship of the two hydroxyl groups can be converted into a weight relationship. The molecular weight of the vinyl alcohol unit is 44, the molecular weight of the non-alcoholyzed vinyl acetate unit is 86, and based on the alcoholysis degree, the average molecular weight of the polyvinyl alcohol unit = molecular weight of vinyl alcohol×alcoholysis degree+molecular weight of vinyl acetate×(1-alcoholysis degree), e.g., the molecular weight of the unit with 80% alcoholysis degree: 44×80%+86×20% = 35.2+17.2 = 52.4; the molecular weight of the unit with 100% alcoholysis degree is: 44×100% = 44; and each average molecular weight unit corresponds to one hydroxyl group. The molecular weight of the small molecule polyhydroxy compound is divided by the number of hydroxyl groups to obtain the molecular weight of each hydroxyl-containing unit, e.g., 30.67 of glycerol and 30.3 of manicol.

Based on the above estimation, when the hydroxyl ratio of the small molecule polyhydroxy compound to the polyvinyl alcohol is 0.1:1, it can be estimated that, for example, the weight ratio of the glycerol to the polyvinyl alcohol having the alcoholysis degree of 80% is 0.059:1; and when the hydroxyl ratio is 1.3:1, the weight ratio of the mannitol to the polyvinyl alcohol having the alcoholysis degree of 100% is 0.9:1. According to the estimation, the inventors test the polyvinyl alcohol compositions mixed with different amounts of the small molecule polyhydroxy compound. It is found that when the hydroxyl ratio of the small molecule polyhydroxy compound to the polyvinyl alcohol is 0.1:1 to 1.3:1, the thermoplasticity and toughness are good, when the hydroxyl ratio is smaller than 0.1:1, the thermoplasticity and toughness are poorer, the thermoplasticity and toughness begin to change after the hydroxyl ratio is more than 0.1:1, the material begins to show the thermoplasticity and toughness which are suitable for the material to be processed into the microneedle by a simple process, and after the hydroxyl ratio is more than 1.3:1, the material gradually loses the needed toughness. According to test data, the performances are better when the hydroxyl ratio is 0.15:1 to 1:1.

According to the above estimation, when the hydroxyl ratio is 0.1:1 to 1.3:1, with regard to the preferred combination of the present disclosure, for example, manicol is correspondingly added, the corresponding weight ratio is about 0.05:1 to 0.9:1, and the rest can be calculated analogically.

The microneedle substrate composition can be in various forms for use, for example, the microneedle substrate composition can be packaged respectively, can be weighed and mixed for use according to the ratio during use, can also be premixed according to the ratio to form a blended powder for use, can be further hot-pressed into a molded material for use, and can be selected according to a preparation method of a microneedle.

Therefore, the present disclosure further provides a strongly hydrophilic blank microneedle. A microneedle substrate is the microneedle substrate composition of the present disclosure, mainly including polyvinyl alcohol and a small molecule polyhydroxy compound, a hydroxyl ratio of the small molecule polyhydroxy compound to the polyvinyl alcohol being 0.1:1 to 1.3:1, the polymerization degree of the polyvinyl alcohol being 300-3,500, and the alcoholysis degree being 80% or more.

Preferably, the polymerization degree of the polyvinyl alcohol is 300-2,600.

Preferably, the alcoholysis degree is 88% or more.

Preferably, the hydroxyl ratio is 0.15:1 to 1:1.

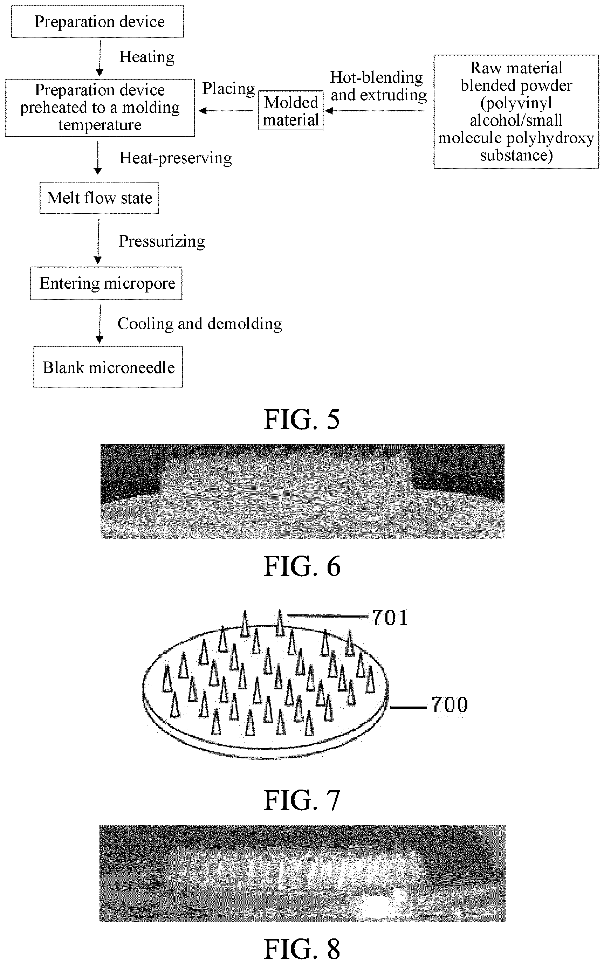

Further, the present disclosure provides a method for preparing a strongly hydrophilic blank microneedle, including: blending the polyvinyl alcohol powder and the small molecule polyhydroxy compound powder according to the hydroxyl ratio (converted into the weight ratio for weighing) at room temperature to form a blended powder, hot-pressing the blended powder into a molded material, and hot-pressing the molded material into a mold to form a blank microneedle; or hot-mixing and melt-extruding the blended powder by a blending machine at a certain temperature into a molded material, and hot-pressing the molded material into a mold to form a blank microneedle (see FIG. 6 and FIG. 7 for details: 700 represents a blank microneedle whole body, and 701 represents a blank microneedle body).

In an optimized example of the present disclosure, the used mold is a flat plate with the thickness of 0.2-3 mm, and has one or more groups of micropore arrays, and the micropore is a through hole. The flat plate is made of metal such as aluminum and copper, and a polymer material. The flat plate made of the materials can be obtained commercially, such as polytetrafluoroethylene, polytrifluoroethylene, polyformaldehyde, polyethere therketone, polyimide and materials obtained by modifying the polymer materials.

When such a mold is used to prepare the substrate composition microneedle, appropriate pressure and temperature are required to obtain a good blank microneedle. Under a certain pressure, the molding temperature is generally positively correlated with the polymerization degree and alcoholysis degree of the polyvinyl alcohol, the molecular weight and melting point of the small molecule polyhydroxy compound, and generally negatively correlated with the ratio of the small molecule polyhydroxy compound.

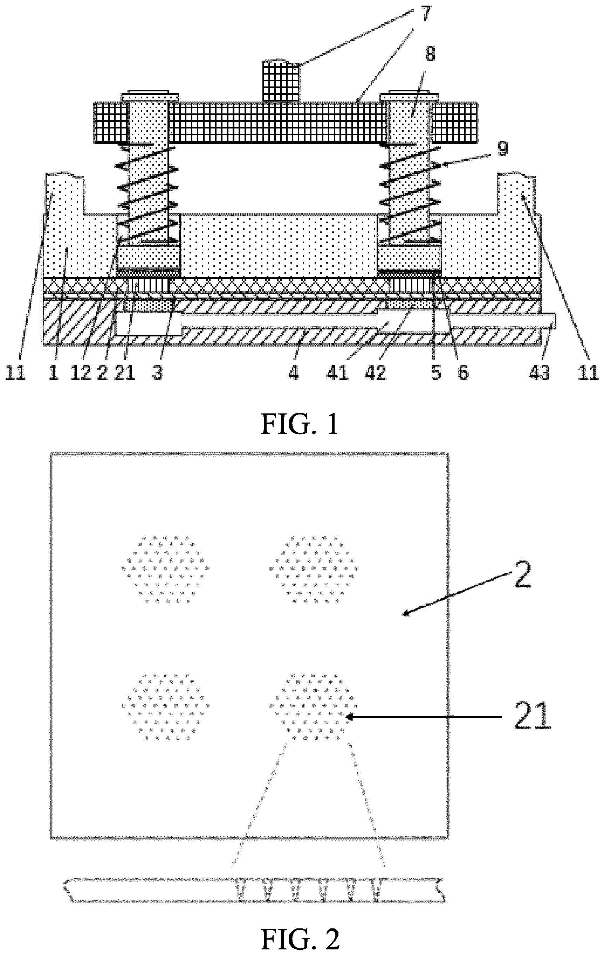

The methods for using the mold: the blended powder or the molded material is added on the mold and heated to form a melt flow material able to flow, a pressure can be applied from an upper part of the material on the mold downwards, or vacuumizing is conducted from a lower part of the mold to reduce pressure, or a pressure is applied from the upper part and vacuumizing is conducted from the lower part at the same time so as to enable the melt flow material to enter the micropores of the mold. A better preparation method is to use a dedicated microneedle preparation device, generally including an upper pressurization and lower depressurization system (details are shown in FIG. 1 and example 2; and references: ZL 2018 2 2070608.8, ZL 2018 2 2068011.X, ZL 2018 2 2070605.4, ZL 2018 2 2071901.6 and ZL 2018 2 2070604.X).

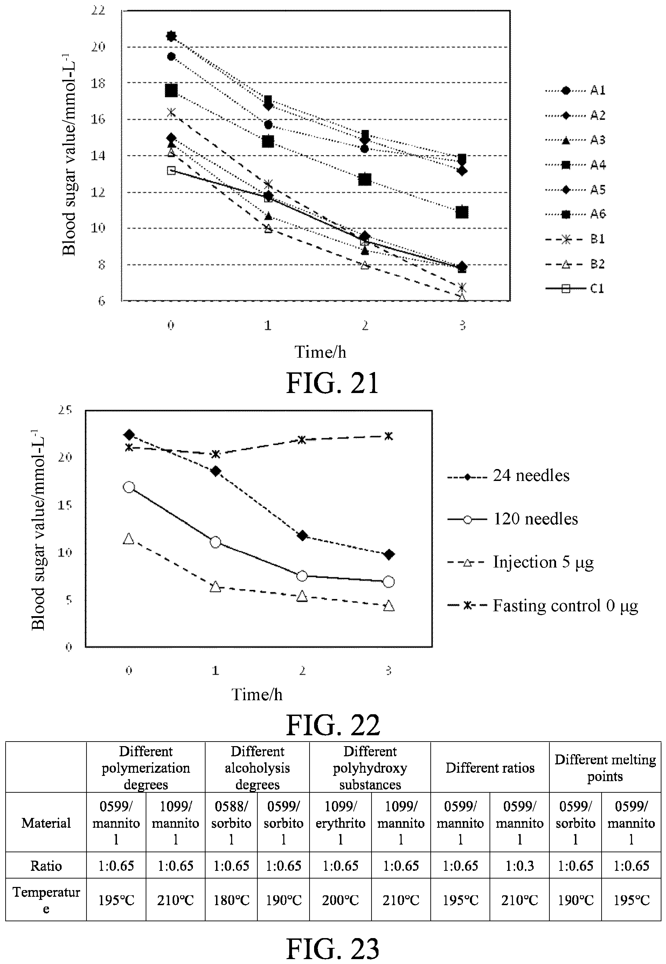

The temperature range is as the example listed in the table of FIG. 23. The lowest molding temperature for manufacturing a blank microneedle from hot-extruded materials of substrate compositions with different components under a certain pressure can be obtained by a test and used as a lower temperature limit of a suitable temperature range, and an upper temperature limit should be lower than the decomposition temperature of the used polyvinyl alcohol. Within the range of the lower limit and the upper limit of the temperature, the blank microneedle can be prepared only by adjusting the pressure and shortening the heating time of the material without special limitation. The contact with oxygen is reduced, which is beneficial to preventing the polyvinyl alcohol from decomposing. For reference, the literature reports that the decomposition temperature is about 240°C of the polyvinyl alcohol with the alcoholysis degree of 88% and about 245°C of the polyvinyl alcohol with the alcoholysis degree of 99% (Jin Sunsen, and tutors Zhong Mingqiang and Fan Ping, "Preparation and Performance Research of PVA with High Decomposition Temperature", Master's Thesis of Zhejiang University of Technology, School of Materials Science and Engineering, January 2019).

The substrate of the present disclosure has good thermoplasticity and toughness and is beneficial for the process stress. The surface of the prepared microneedle has strong hydrophilic polarity and is beneficial for the firm attachment of a drug, and the drug-loading capacity is increased.

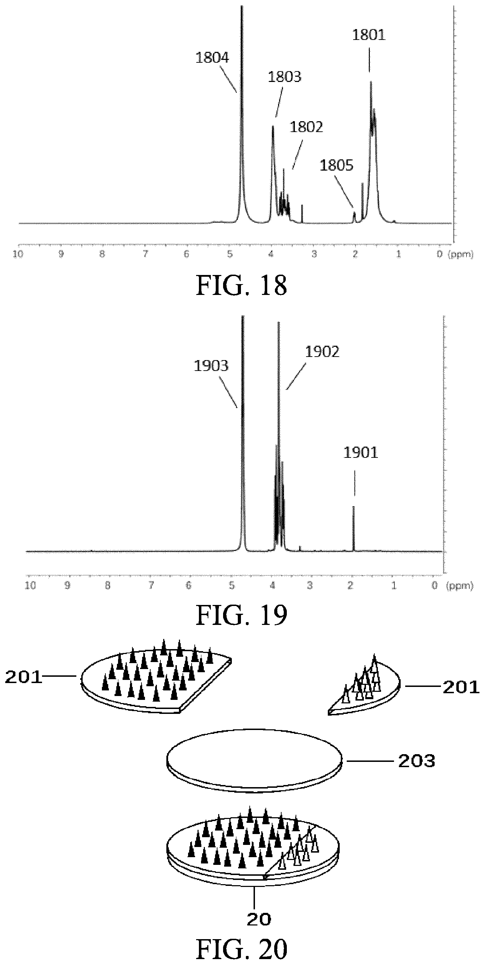

By utilizing the blank microneedle, the present disclosure also provides a drug-loaded microneedle formed by contacting and attaching a solution containing a drug with the blank microneedle and drying the microneedle. As long as the drug can be adsorbed on the strongly hydrophilic surface, the drug-loaded microneedle can be formed by contacting the blank microneedle prepared in the present disclosure with the prepared solution containing a drug for adsorption and loading, and drying the microneedle. Therefore, the microneedle can be widely used in drug administration as required. Particularly, the blank microneedle of the present disclosure can be conveniently used for loading a drug which cannot tolerate a high temperature. The drug-loaded microneedle can form drug enrichment on the skin layer of the drug administration part, which is beneficial to the treatment of related diseases. The oral administration or injection of the drug needs to pass through a digestion and absorption system and/or be distributed in the whole blood circulation. The drug administration efficiency of local skin is not high. The drug is applied outside the skin, and thus is separated by the epidermis and has an insufficient permeation. For example, it is known that finasteride can relieve alopecia and promote hair growth, preferably the drug is used in a small dosage and concentrated on the superficial layer of the scalp. Through the microneedle administration, the drug can be enriched on the scalp in a small dosage, which reduces the harmful effect on the whole body and has obvious advantages. For another example, when the insulin microneedle is used for administration, the microneedle is only needed to be pasted on the skin, no injection is needed, and pain is reduced. Such a microneedle can further be easily made into a combined microneedle with several drugs respectively loaded on needle tips of different parts to combine several drugs into a whole, so as to reduce the administration times. For example, the insulin microneedle is made into a circular big half part, the exenatide microneedle is made into a circular small half part, the circular backs of the two parts are bonded on another wafer to form a combined microneedle (see FIG. 20: 20 is a full needle, 201 is a microneedle large semicircle loading one drug, 202 is a microneedle small semicircle loading the other drug, and 203 is a back plate for bonding). The microneedles loaded with different drugs are mutually separated and also spliced together to facilitate use.

The tip of the blank microneedle can load many kinds of drugs, such as an insulin drug, an exenatide drug, human growth hormone, finasteride, a calcitonin drug, a protein drug and a peptide drug. The basic principles for selecting a drug are proper dosage, other administration methods being less convenient than microneedles, the need to form an enrichment concentration of a drug on the local skin, instability of the drug in other preparation forms, and the like. Due to the two outstanding pharmaceutics advantages of the microneedle preparation technology of the present disclosure: the drug is not subjected to a high temperature and the coated drug-loading capacity is larger, and the common advantages of the microneedle preparation, the selected drug is proper as long as one or more of the advantages can be realized. The proper selection can be a single drug or the compound compatibility of two or more drugs which are mixed and contacted. The compound compatibility of drugs is that the quality is not influenced after several drugs are mixed and contacted, and the drug effect can be exerted. Preferably, it should be approved by medical regulations in other preparation forms, such as the injection "Novolin" where short-acting insulin is combined with intermediate-acting insulin. With regard to a drug whose quality will be affected by a direct contact, if it is needed to be administered simultaneously, the combined microneedle is an ideal. Therefore, there is no need to make any special restrictions on the drug species, and it is not necessarily to lose the intellectual property protection of the microneedle preparation of the present disclosure of a suitable drug species due to no special restrictions either.

The drug-loaded microneedle is formed by contacting and attaching a blank microneedle and a drug-containing solution, and drying the microneedle, namely, the preparation of the blank microneedle and the drug-loading process of the microneedle are conducted in stages. Such a blank microneedle can be conveniently produced in a large scale. The convenience lies in: I, only the preparation of the blank microneedle is conducted at a high temperature, the conditions of the subsequent drug loading are very mild, and the drug quality is ensured; II, the drug is not mixed with the microneedle material into a whole, the properties of the microneedle material are not influenced, and the factors such as the toughness and the brittleness of the blank microneedle material are easy to regulate; III, the adjustment of the drug release behavior can be conveniently realized only by adjusting auxiliary materials in the drug liquid; and with regard to the mixed drug-loaded microneedle, both the properties of the material and the release behavior of the drug should be considered, and thus the regulation difficulty and related factors are increased; and IV, the drug liquid is only wrapped on the surface of the needle tip to form a liquid film with the large surface area and small thickness, the drying time is short, the temperature is low, and the process amplification is very facilitated.

As long as the drug-containing solution is beneficial to loading of the drug, does not influence the exertion of the drug effect, and does not produce adverse effects on human bodies, common related pharmaceutic auxiliary materials can be used to help the loading of the drug and better exert the drug effect.

Further, a method for drug loading by the blank microneedle prepared by the present disclosure is characterized in that a water-soluble drug is directly prepared into an aqueous solution for drug loading, and a proper amount of a suitable adhesive is better added for drug loading; if a solid film layer formed by drying the water-soluble drug has a satisfactory adhesive force with a microneedle substrate, does not shrink to generate cracks, and does not generate a shearing force on the bonding interface of the drug and the microneedle, the solid film layer can be firmly attached to the surface of the needle tip, and thus an adhesive is generally not added or slightly added; a water-insoluble drug is uniformly suspended in water or dissolved/suspended in a non-aqueous polar solvent for drug loading, and a proper amount of an adhesive is better added for drug loading; and the non-aqueous polar solvent is typically methanol, ethanol, and isopropanol, mostly commonly ethanol, in view of polarity, solubility, and biosafety. In specific use occasions, for example, where rapid release is needed, auxiliary materials with strong water solubility and proper viscosity can be used. The proper viscosity means that the drug can be bonded on the microneedle and the auxiliary material can be rapidly dissolved to promote the drug to rapidly contact body fluid, and generally auxiliary materials of low-molecular-weight povidone or polyethylene glycol can be selected. If slow and long-acting release is required, povidone or polyethylene glycol with different molecular weights can be generally compared and selected. Along with the increase of the molecular weight, the hydrophilicity of the auxiliary materials is kept unchanged, but the viscosity is increased. After the auxiliary materials are in contact with body fluid, the swelling process and the process of dissolving into the body fluid after the swelling are both slowed, and the drug release is also slowed. The dosage of the auxiliary materials is closely related to the drug release behavior. The drug release can also be adjusted by adjusting the amount of the auxiliary materials on the premise of ensuring the firm attachment. If an auxiliary material does not meet requirements on both viscosity and water solubility, related substances can be further added to change the viscosity or water solubility. In the aspect of pharmaceutics, there are many methods for changing the drug release behavior and available auxiliary materials. The drug release behaviors can be compared and selected through tests, so as to achieve the balance of factors such as the drugs, the auxiliary materials, the adhesion and the drug release. In certain use occasions, for example, where rapid release and long-acting effect maintaining are needed, two drugs of rapid release and slow release are mixed together into a microneedle (for example, the injection "Novolin" where short-acting insulin is combined with intermediate-acting insulin) or prepared into the combined microneedle. Therefore, the selection of auxiliary materials and the dosage ratios which are beneficial to drug loading and release on the microneedle of the present disclosure both belong to the concept and the protection scope of the present disclosure.

The hydrophilicity and hydrophobicity of the drug can be improved by methods such as forming a salt, forming a base, exchanging an acid group or a basic group and adjusting a pH value of the drug liquid, as long as the drug effect and the biologically acceptable form are facilitated. For example, the hydrophilicity and hydrophobicity of the polypeptide protein drug are greatly changed near an isoelectric point, and the drug can be prepared and used as required.

In an optimized group of examples of the present disclosure, an aqueous solution of the water-soluble drug is a sodium chloride solution (30% w/w), where sodium chloride is used as a simulated water-soluble drug; an adhesive is povidone k30 (or polyethylene glycol 600) and is prepared into a 3%-15% aqueous solution; and a water-insoluble simulated drug is calcium carbonate powder (200-mesh), at this time, the selection of the concentration of the adhesive solution should also consider the appropriate specific gravity, for example, 8%-13% of povidone k30, the solution is shaken and mixed uniformly, and stood for 2 hours, and calcium carbonate powder is still uniformly suspended in the solution and does not settle or float. The two solutions are placed in an open manner for 10 minutes in an environment with the temperature of 25°C and the relative humidity of 85%, and the weight of the solutions is reduced by less than 1% by volatilization to meet the requirement on the accuracy of drug loading.

In a group of examples of the present disclosure, a povidone k30 aqueous solution (10% w/w) is used, insulin powder and the povidone aqueous solution are mixed into a solution (20:80 w/w) containing insulin, the solution is in contact with a blank microneedle to be loaded to prepare an insulin microneedle, and the insulin microneedle is pasted to the ear of a model pig of diabetes, and has a remarkable effect on reducing blood sugar.

In a group of examples of the present disclosure, a povidone k30 aqueous solution (10% w/w) is used, exenatide powder and the povidone aqueous solution are mixed into a solution (2:98 w/w) containing exenatide, the solution is in contact with a blank microneedle to be loaded to prepare an exenatide microneedle, and the exenatide microneedle is pasted to the ear of a model pig of diabetes, and has a remarkable effect on reducing blood sugar.

In short, when the solution containing a drug is prepared, the loading amount and the adsorption strength can be confirmed by tests, and an additive that contributes to loading and increasing or reducing an adsorption force can be added for adjusting. The additive can be a substance that adjusts pH, viscosity, ionic strength, film forming property, and the like.

The preparation of the microneedle of the present disclosure can be realized by hot-pressing the blended powder into a molded material (wafer) and then hot-pressing the molded material into a microneedle, namely adding the blended powder into a dedicated wafer manufacturing device, putting the device in a hot environment to be heated to a hot-pressing temperature (for example, 0599/mannitol powder (1:0.65 w/w) and 200°C), cooling the material to room temperature after hot pressing for a certain time, and taking out the prepared wafer; and then putting the wafer into a blank microneedle manufacturing device, putting the device in a hot environment to be heated to a hot-pressing temperature (for example, 0599/mannitolpowder (1:0.65 w/w) and 200°C), cooling the material to room temperature after hot pressing for a certain time, taking out a mold plate, and separating a microneedle from the mold to obtain a blank microneedle, preferably, processing the material thermally extruded by the device into the molded material (wafer), putting the wafer into the dedicated blank microneedle manufacturing device, putting the device in a hot environment to be heated to a hot-pressing temperature for several minutes, cooling the material to room temperature after hot pressing for a certain time, taking out a mold plate, and separating a microneedle from the mold to obtain a microneedle, further, in order to reduce the heating time of the wafer, the dedicated device can be firstly preheated to a pressing temperature, the prefabricated wafer is placed on the mold preheated to the required temperature, the temperature is kept for several minutes, the wafer reaches the hot-pressing temperature and is pressurized for several minutes, the mold plate is taken out when it is hot, and cooled, and the microneedle is separated from the mold to obtain the microneedle (details are shown in FIG. 1 and example 2; and references: ZL 2018 2 2070608.8, ZL 2018 2 2068011.X, ZL 2018 2 2070605.4, ZL 2018 2 2071901.6 and ZL 2018 2 2070604.X).

The composition of the substrate and the blank microneedle of the present disclosure can be identified by a spectrum method, for example, characteristic peaks of nuclear magnetic resonance spectrums and the like are used for characterizing the composition formed by the polyvinyl alcohol and the small molecule polyhydroxy compound, and a qualitative detection can be conducted by a specific chromogenic reaction of the polyvinyl alcohol and the small molecule polyhydroxy compound, and divalent cupric ions. The properties can be evaluated by testing the hydrophilicity, adsorptivity, resistance to mechanical peeling, etc. For example, a test solution containing sodium chloride, povidone k30, and water is prepared, a hydrophobic polystyrene wafer, a hydrophilic ethylene-vinyl alcohol copolymer wafer, and a strongly hydrophilic polyvinyl alcohol/small molecule polyhydroxy compound wafer of the present disclosure are prepared, the test solution is respectively dropped on the wafers, and after the wafers are left to dry, the adhesion condition is observed. The substrate wafer of the present disclosure firmly adsorbs solid spots of the test solution, the solid spots cannot be peeled off by a pin, and only scratches are remained on the surface. The solid spots are not blown off by an electric blower. The spots on other control wafers are easily peeled off and blown off.

For example, the characteristic presence of polyvinyl alcohol and a small molecule polyhydroxy compound can be detected by using a chromogenic reaction and hydrogen proton nuclear magnetism after the blank microneedle of the present disclosure is crushed and pretreated.

The prepared drug-loaded microneedle can be identified by the same method as that of the blank microneedle. If the loaded drug has an influence on the identification, the tip of the drug-loaded microneedle can be cut off first and then the microneedle is identified by the same method.

In the manufacturing of the blank microneedle of the present disclosure, there are also the process items of convenient operation such as the separation of the microneedle from the mold plate and the removal of residual materials in micropores of the mold. When the ratio of the small molecule polyhydroxy compound to the polyvinyl alcohol is less than 0.15:1, the brittleness of the substrate is high, such that the separation difficulty is increased and the microneedles often break. After the ratio is greater than 0.15:1, the microneedles can be relatively easily separated and the number of the microneedles is kept complete. A small amount of materials sometimes remain at the tips of the micropores of the mold after the microneedle separation and need to be removed. Otherwise, the micropores will be blocked, and the mold is disabled. The best method for removing the residue is to boil and dissolve out the material in water. However, when the polymerization degree of the polyvinyl alcohol is greater than 2,600, the boiling time is obviously too long, since when the polymerization degree is greater than 2,600, although the material can be fully swelled, the viscosity is too high, the time for dissolving the material into a water phase is too long, and the amplification of the mass production process is not facilitated. In addition, the solubility of the polyvinyl alcohol with the polymerization degree of 78%-85% is the largest in cold water. After the needle body of such a polymerization degree enters the skin hole to absorb body fluid, the needle body excessively swells and is subjected to disordered deformation, such that the drug release behavior is greatly changed. However, such a phenomenon is not observed in the polyvinyl alcohol with the polymerization degree of 88% or more.

### Example 1: testing and control of molding temperature range of microneedle composition substrate

According to the microneedle composition substrate of the present disclosure, a small molecule polyhydroxy compound and polyvinyl alcohol were blended according to a ratio to obtain a blended powder. On a microneedle preparation device of example 2, in a certain pressure range (28-32 kg/cm²), in order to obtain a better prefabricated wafer and/or microneedle molding, a temperature was adjusted for a test to obtain a lower limit temperature with better operability. The result was shown in the table of FIG. 23. The table showed the molding temperature of a prefabricated wafer and/or microneedle of different hot-extruded materials under the pressure condition of 28-32 kg/cm², where the ratio is the weight ratio, and the unit is w/w.

The thermoplastic temperature of the polyvinyl alcohol was far below its decomposition temperature after the small molecule polyhydroxy compound such as manicol, sorbol, and erythritol was added. The literature reported that the decomposition temperature is about 240°C of the polyvinyl alcohol with the alcoholysis degree of 88% and about 245°C of the polyvinyl alcohol with the alcoholysis degree of 99% (Jin Sunsen, and tutors Zhong Mingqiang and Fan Ping, Preparation and Performance Research of PVA with High Decomposition Temperature, Master's Thesis of Zhejiang University of Technology, School of Materials Science and Engineering, January 2019).

### Example 2: preparation device and process method for prefabricated wafer, blank microneedle, and wafer of other materials for test

FIG. 1 was a schematic diagram of an example of the microneedle preparation device used in the present disclosure. A test blank microneedle and a test sample wafer of the present disclosure were both prepared by the device.

As shown in FIG. 1, the dedicated microneedle preparation device successively includes an upper pressing plate 1, a mold plate 2, an isolation layer 3, and an air extraction plate 4 from top to bottom. The isolation layer 3 is made of an air-permeable and liquid-impermeable material. The mold plate 2 is provided with micropore areas 21 corresponding to needle-shaped objects of microneedles, the micropores are through holes, a cavity 41 is arranged in the air extraction plate 4, air extraction micropores 42 communicating with the cavity 41 are arranged in the air extraction plate 4 corresponding to the micropore areas 21, an air extraction pipe 43 communicating with the cavity is arranged outside the air extraction plate 4, an auxiliary pressing device 11 manually or automatically lifted upwards and pressed downwards is arranged above the upper pressing plate 1 (the auxiliary pressing device 11 is not shown in detail in the figure), so as to conveniently press downwards and loosen upwards the mold, the upper pressing plate 1 is also provided with through holes 12, and the positions of the through holes 12 are consistent with those of the micropores 21 of the mold plate for feeding and pressurizing, and limiting the tablet diameter. The positions of the through holes 12 in the upper pressing plate 1 are further consistent with those of pressure rods 8 on another auxiliary pressure device 7 (the auxiliary pressure device 7 is not shown in detail in the figure) above, and through the through holes 12, the pressure device 7 above is manually or electrically lifted upwards or pressed downwards to press and separate a microneedle. The pressure rods 8 apply a pressure to a melt flow substrate through springs 9 sleeved thereon, and the springs 9 are used for controlling the downward pressure within a certain range. Isolation layers 5 are arranged between the pressure rods 8 and microneedle materials 6 to prevent mutual adhesion and facilitate separation. The pressure rods 8, the through holes 12, and the auxiliary pressure device 7 are in a sliding tight fit relationship, so as to ensure positioning, sliding, and no overflow of materials.

An operation process is as follows: the upper pressing plate 1 is descended and the mold plate 2 is pressed; the pressure rods 8 are lifted to leave the through holes 12, a molded material is placed on the micropore areas 21 of the mold through the through holes 12, and the pressure rods 8 are descended to press the molded material into a microneedle; the pressure rods 8 are lifted to release the pressure of the springs 9 on the material; the pressing plate 1 is lifted to release the pressing on the mold plate 2, and the pressure rods 8 are lifted to separate from the microneedle; the mold plate 2 is drawn out and cooled, the microneedle 6 on the mold plate is separated; and all the lifting and descending are realized by the auxiliary pressing device 11 and the auxiliary pressure device 7. When a wafer is prefabricated, the mold plate 2 is only replaced by a flat plate made of the same material. The springs 9 are commercially available molded green rectangular springs with the designated pressure of 32 kg/cm², and are discarded after the pressure is reduced to 28 kg/cm² by repeated use, such that the pressure range is set to be 28-32 kg/cm². Hereinafter, this spring is referred to unless otherwise specified. Similarly, the relationship between the pressure and the temperature may vary within a certain range, and is not strictly limited unless otherwise specified.

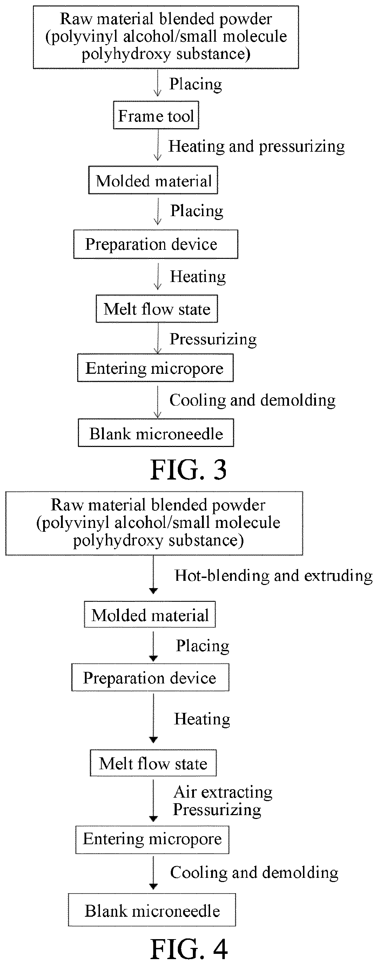

The process for manufacturing the microneedle by the device was simple (see the flow of FIG. 3 and the flow of FIG. 4), and the operation process was as described above: a preprepared molded material (a wafer formed by hot pressing a blended powder or a wafer or a particle material extruded by a hot-blending machine) was placed in a device, the whole device was heated to a hot-pressing temperature (the temperature was determined according to the substrate and example 1 and the temperature range is generally between 180°C and 225°C), the temperature was kept (10 minutes) to enable the temperature of the device to be uniform, the air extraction system 4 and the pressure system 7 were started to extrude a melt flow material into the micropores to prepare a microneedle, and the whole device was taken out to be cooled to obtain a blank microneedle. The photo of the blank microneedle was shown in FIG. 6. It can be seen that the surface of the blank microneedle was smooth and free of burr, and the shape and the size were uniform. The structure of the blank microneedle was shown in FIG. 7, and in FIG. 7, 700 was a whole blank microneedle, and 701 was a blank microneedle body.

Preferably, the operation process of preparing the microneedle by the device was as follows (see the flow of FIG. 5): the device was always placed in a hot-pressing temperature environment (an oven; and the temperature range was about between 180°C and 220°C according to the properties of the substrate material), and after the temperature was reached for 10 minutes, the heat inside the device was balanced; the substrate wafer was placed into the device, the temperature was kept for 2 minutes, the wafer reached the temperature, air extraction and pressing were conducted for 1 minute to prepare the microneedle; and the mold plate with the microneedle was withdrawn and cooled to obtain a blank microneedle. In the example, the heating and cooling time of the whole device was saved by 45 minutes to 55 minutes, the wafer was heated for only 2 minutes and pressed for 1 minute, the heat capacity of the withdrawn mold plate and the microneedle thereon was small, the cooling speed was high, the heating time of the substrate was greatly shortened, and the quality assurance and the process amplification of the microneedle were facilitated.

The simple operation process of the wafer manufacturing was very similar to that of the blank microneedle, and only a base plate made of the same material was used for replacing the mold plate to obtain the wafer.

### Example 3: adhesion test of substrate

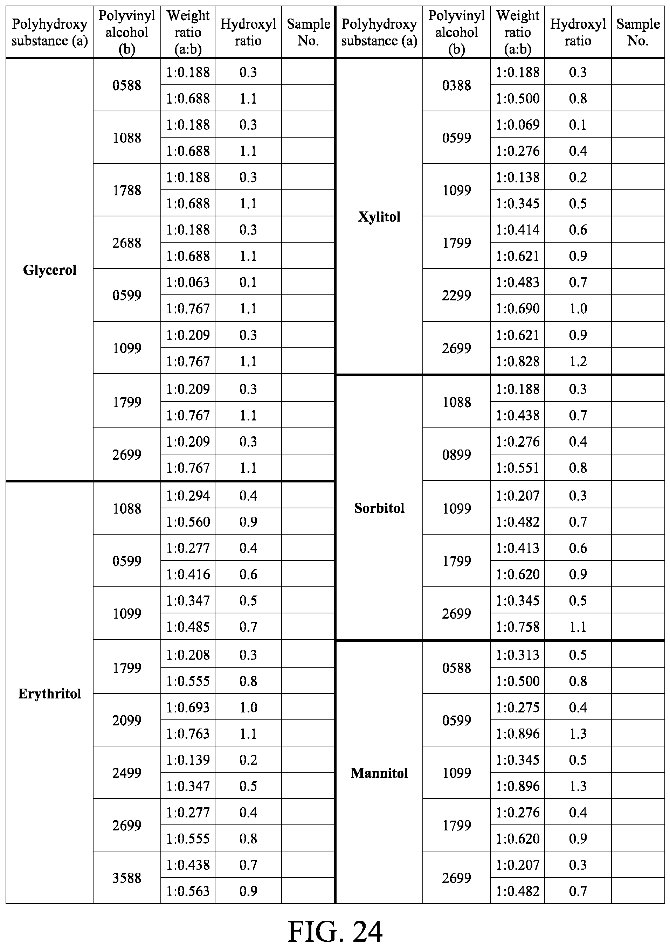

Preparation of three test wafers: (1) hydrophobic polystyrene wafer (powder of Dongguan Xingwang Plastic Raw Materials Co., Ltd., no indication of molecular weight, but no hinder of determination of adhesion); (2) hydrophilic ethylene-vinyl alcohol copolymer wafer (three powders of Kuraray, Japan: E105B (44 mol/56 mol), F104B (32 mol/68 mol), and L171B (27 mol/73 mol), no indication of molecular weight, but no hinder of determination of adhesion); and (3) strongly hydrophilic polyvinyl alcohol/small molecule polyhydroxy compound wafer of the present disclosure (polyvinyl alcohol powder of Shanghai Yingjia Industrial Development Company, and each small molecule polyhydroxy compound are purchased from several manufacturers), the polyvinyl alcohol/small molecule polyhydroxy compound substrate wafer was shown in the table of FIG. 24 for details.

Preparation of test solution: solution consisting of sodium chloride, povidone k30, and water at a weight ratio of 20/8/72. The solution was placed in an open manner for 10 minutes in an environment with the temperature of 25°C and the relative humidity of 85%, and the weight of the solution was reduced by less than 1% by volatilization to meet the requirement on the accuracy of drug loading.

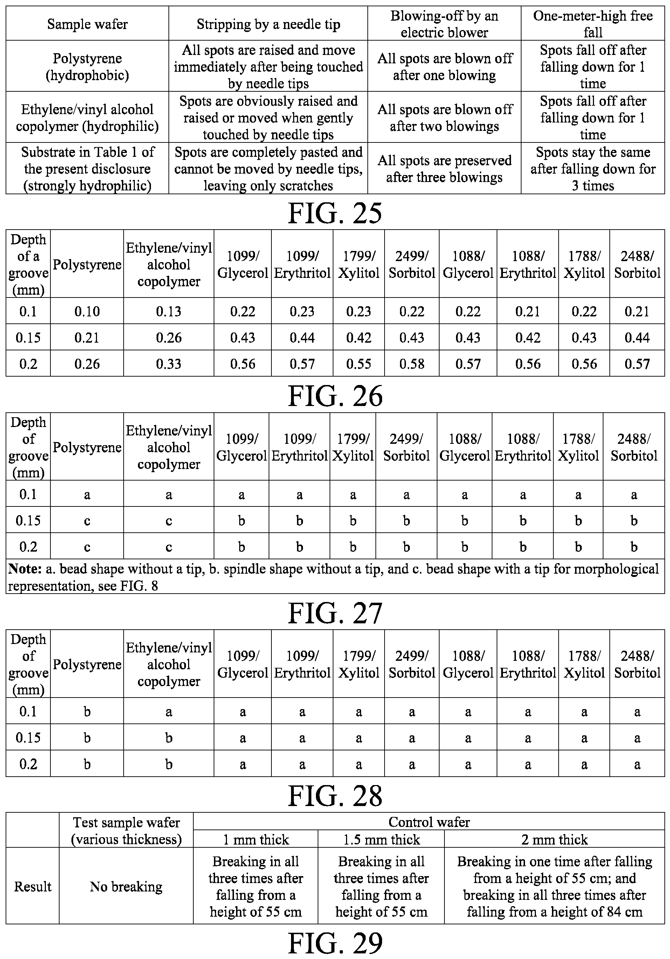

A small amount (about 0.1 mg) of the test solution was respectively dipped on the surface of the wafer, and the wafer dipped with drug liquid spots on the surface was placed in a dryer at room temperature for 24 hours to obtain a spotted wafer. The following test observation was conducted. The result of the adhesion test of the substrate to the spots formed by the sodium chloride/povidone solution was shown in the table in FIG. 25. The result showed that the substrates of the present disclosure all showed very good adsorption fastness.

Manual needle tip stripping test: under a high power magnifying glass (30x), the spots were touched by a metal needle tip, and the changes of the spots were observed.

Blowing off test of electric blower: the spots faced upwards and were pasted on the wafer and blown cool by an electric blower for 3 minutes, and whether the spots were blown off or not was observed by a high power magnifying glass (30x).

One-meter-high free fall test: after each wafer freely fell through a one-meter-long glass tube, whether the spots fell off or not was observed by a high power magnifying glass (30x).

### Example 4: hot-pressing blended powder substrate into wafer and wafer into microneedle

According to the method described in example 2 above, the dedicated manufacturing device in FIG. 1 was used, the flow of FIG. 3 was followed, 0.65 g of a blended powder (1:0.65 w/w) of polyvinyl alcohol 0599 and mannitol powder was added into the dedicated manufacturing device, the device was placed in a hot environment to be heated to a hot-pressing temperature (200°C), pressurized and heat-preserved for 27 minutes, and cooled to room temperature to obtain a wafer; and the wafer was placed in the microneedle manufacturing device, and the device was heated to a hot-pressing temperature (200°C), pressurized and heat-preserved for 27 minutes, and cooled to room temperature to obtain a blank microneedle.

### Example 5: hot-pressing hot-blended and extruded material into wafer and wafer into microneedle

According to the method described in example 2 above, the dedicated manufacturing device in FIG. 1 was used, the flow of FIG. 4 was followed, 0.65 g of a mechanically hot-extruded particle material of a blended powder (1:0.65 w/w) of polyvinyl alcohol 0599 and mannitol powder was added into the dedicated manufacturing device, the device was placed in a hot environment to be heated to a hot-pressing temperature (200°C), pressurized and heat-preserved for 27 minutes, and cooled to room temperature to obtain a wafer; and the wafer was placed in the microneedle manufacturing device, and the device was heated to a hot-pressing temperature (200°C), pressurized and heat-preserved for 27 minutes, and cooled to room temperature to obtain a blank microneedle.

### Example 6: manufacturing of wafer and blank microneedle according to method for shortening heating time of material

According to the method for shortening the heating time of the material described in example 2, the dedicated manufacturing device in FIG. 1 was used, the flow of FIG. 5 was followed, the dedicated manufacturing device was preheated to a hot-pressing temperature (200°C), after the whole temperature of the device was balanced for 10 minutes, 0.65 g of a blended powder of polyvinyl alcohol 0599 and mannitol powder (1:0.65) or 0.65 g of a mechanically hot-extruded particle material with the same ratio was taken and added into the dedicated manufacturing device, the material was continuously heated for 2 minutes (to enable the material to reach the temperature) and hot-pressed for 1 minute, the pressure was relieved, and the base plate was drawn and cooled to obtain a wafer; the dedicated manufacturing device was preheated to a hot-pressing temperature (200°C), after the whole temperature of the device was balanced for 10 minutes, the wafer was placed into the device and continuously heated for 2 minutes (to enable the wafer to reach the temperature) and hot-pressed for 1 minute, the pressure was relieved, and the mold plate was drawn and cooled to obtain a blank microneedle.

### Example 7: microneedle drug-loading capacity test, drug liquid adhesion form observation and analysis, and adhesion fastness test

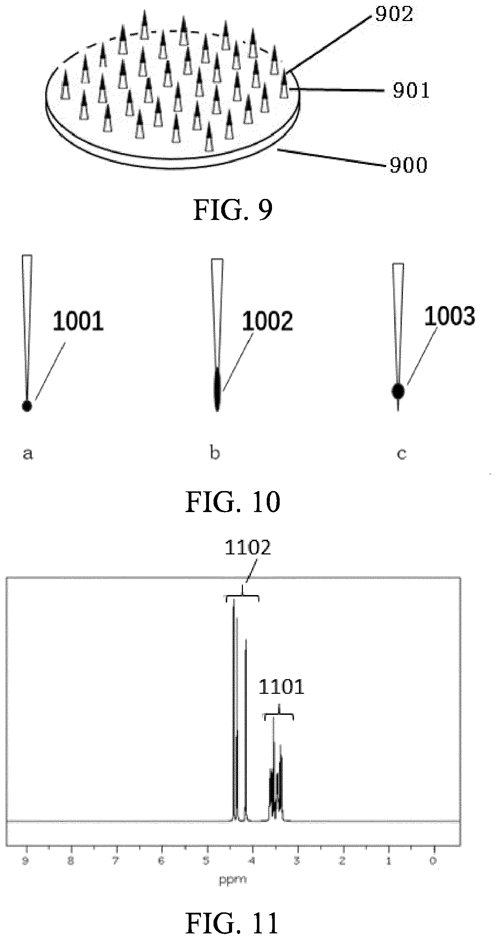

A solution consisting of sodium chloride, povidone k30, and water at a weight ratio of 20:8:72 was used in a blank microneedle drug-loading capacity test. After the blank microneedle loaded a drug, the image of the drug firmly adsorbed at a needle tip position under a high power magnifying glass (30x) was shown in FIG. 8. The structure of the drug-loaded microneedle was shown in FIG. 9. In FIG. 9, 900 was a whole drug-loaded microneedle, 901 was a drug-loaded microneedle body, and 902 was a drug-loading part of tips of drug-loaded microneedle bodies.

Test of blank microneedle adsorption of test solution: a shallow circular groove with a diameter of 30 mm was formed in the surface of a square metal aluminum block (5 cm×5 cm×2 cm) with the depths of 0.1 mm, 0.15 mm, and 0.2 mm, respectively, and a test solution with a limited solution height was obtained by feeding the test solution into the groove and scraping off overflown liquid by translation with a scraper. The blank microneedles made of various materials according to the ratio were precisely weighed (1/100,000 scale), the needle tips faced downwards vertically and stood in the circular groove for 3 seconds, the microneedles were taken out and precisely weighed, and net weight gain was calculated by weight loss; the form of the drug liquid drop hanging on the needle tip was observed by a high power magnifying glass (30x); and the drug-loaded microneedle was hung in a dryer (room temperature for 24 h) and then freely fell down by one meter, and the falling-off condition of the needle tip drug was observed by a high power magnifying glass (30x). The results were shown in the tables in FIG. 26 to FIG. 28.

FIG. 26 showed the amount of the test solution adsorbed by the microneedle in mg; and FIG. 27 showed the state of the drug liquid drop hanging on the needle tip observed by a high power magnifying glass (30x), where a represented a bead shape without a tip, b represented a spindle shape without a tip, and c represented a bead shape with a tip. The three forms were shown in FIG. 10.

When the microneedle left the liquid level, the form of the liquid drop hanging on the needle tip was immediately observed by a 30x magnifying glass. The state of the needle tip wrapped by the drug liquid can be seen in FIG. 10. which presents hanging the drug liquid on the blank microneedle of different substrates. When the liquid hanging amount was small enough, the liquid hanging on the materials was wrapped on the needle tip in a bead shape as a in FIG. 10, state 1001 of a bead shape without a tip. Along with the increase of the liquid hanging amount, the liquid on the tip of the strongly hydrophilic substrate microneedle of the present disclosure was spread into a spindle shape along an axial direction and wrapped the needle tip, as b in FIG. 10, state 1002 of a spindle shape without a tip, indicating that the affinity between solid and liquid phases was greater than the liquid-phase surface shrinkage force. In comparison, the liquid on the needle tip of the substrate, i.e., the weakly polar or non-polar material, moved upward in the form of a bead shape to expose the needle tip, as c in FIG. 10, state 1003 of a bead shape with a tip, indicating that the affinity between solid and liquid phases was smaller than the liquid surface shrinkage force.

It can be reasonably presumed that when the microneedle is inserted, the microneedle made of the strongly hydrophilic substrate of the present disclosure can bring the strongly attached drug into the skin hole, while the weakly attached drug of the control microneedle will be partially pushed out of the skin. The specific drug effect is also deduced in the subsequent drug effect example test.

Due to the depth limitation of the microneedle entering the skin, generally only the drug wrapped in the needle tip within a length of 0.3 mm can stably generate the drug effect, and the drug in the rear part was gradually far away from the most easily absorbed section, such that wrapping by the b-shaped spindle-shaped drug liquid without the tip was a better state.

FIG. 28 is the table showing falling-off of the drug observed by a high power magnifying glass (30x) after the drug-adsorbed microneedle freely fell off by one meter, where in FIG. 28, a indicated no falling off and b indicated falling off.

### Example 8: anti-impact toughness test of microneedle substrate

A wafer having a diameter of 1.9 cm, and a thickness of 1 mm, 1.5 mm, and 2 mm was prepared according to the substrate of the present disclosure in example 3 and was used as a test sample. Povidone (PVP k-30) was prepared into a wafer of the same size as a control.

The wafers of the test sample and the control were respectively laid flat on a steel plate in an environment of 17°C and the relative humidity of 46%, and freely fell on wafer needles vertically through a glass tube of a 2 cm inner diameter and a 55 cm or 84 cm length using 15.8 g of a steel cylinder. The operation was repeated 3 times. The breaking condition was shown in the table of FIG. 29.

The reasons for selecting the povidone wafer as the control test sample were: 1. the wafer can be thermoplastically molded; and 2. as the most common substrate for many cases of microneedles, the toughness can substantially tolerate the subsequent process stress, and the test result was shown in the above table. The substrate of the present disclosure had a better toughness and was more suitable for microneedle processing.

### Example 9: blank microneedle drug-loading test

When the blank microneedle prepared according to the present disclosure was used for loading a drug, generally a water-soluble drug was directly prepared into an aqueous solution for drug loading, or a proper amount of an adhesive was added for drug loading. If a solid film layer formed by drying the water-soluble drug had a satisfactory adhesive force with a microneedle substrate, did not shrink to generate cracks, and did not generate a shearing force on the bonding interface of the drug and the microneedle, the solid film layer can be firmly attached to the surface of the needle tip, and thus an adhesive was generally not added or slightly added. A water-insoluble drug was uniformly suspended in water or dissolved/suspended in a non-aqueous polar solvent for drug loading, and a proper amount of an adhesive was better added for drug loading.

The same test was conducted using the square metal aluminum block (5 cm×5 cm×2 cm) in example 7.

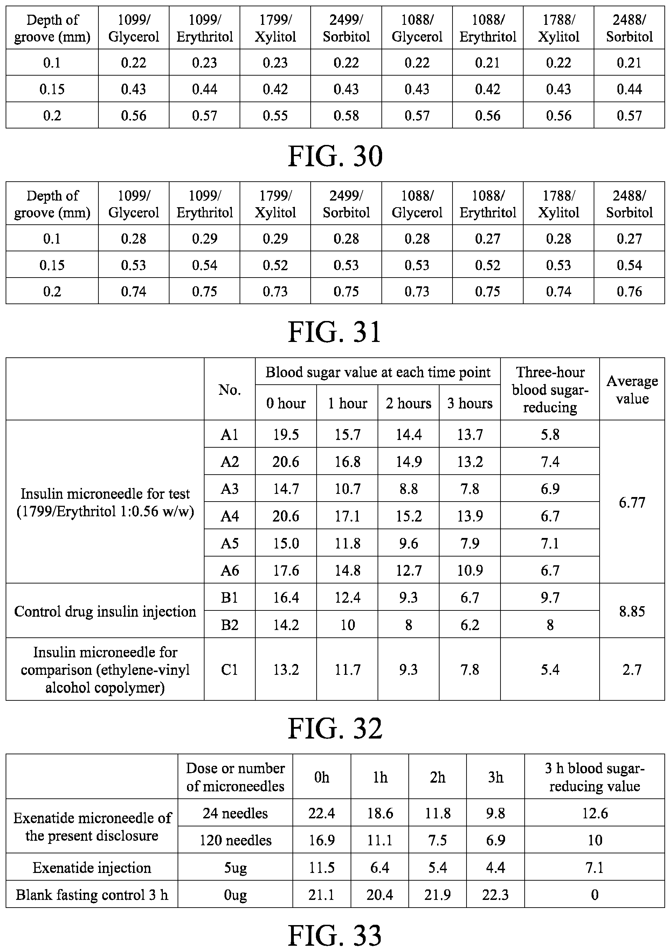

In a loading test of the water-soluble drug, a solution consisting of sodium chloride, povidone k30, and water at a weight ratio of 20:8:72 was used as a test solution. The solution was placed in an open manner for 10 minutes in an environment with the temperature of 25°C and the relative humidity of 85%, and the weight of the solution was reduced by less than 1% by volatilization to meet the requirement on the accuracy of drug loading. The drug-loading result was shown in the table of FIG. 30. The table showed the amount of test solutions of water-soluble materials adsorbed by microneedles in mg.

In a loading test of the water-insoluble drug, a used test solution was a suspension formed by mixing 20 g of calcium carbonate powder (200-mesh) and 80 ml of a povidone k30 aqueous solution (11%). After the suspension was shaken and mixed uniformly, and stood for 2 hours, no calcium carbonate powder was settled or floated. The solution was placed in an open manner for 10 minutes in an environment with the temperature of 25°C and the relative humidity of 85%, and the weight of the solution was reduced by less than 1% by volatilization to meet the requirement on the accuracy of drug loading. The drug-loading result was shown in the table of FIG. 31. The table showed the amount of test solutions of water-insoluble materials adsorbed by microneedles in mg.

### Example 10: chromogenic detection of microneedle

The specific presence of polyvinyl alcohol and a polyhydroxy compound in the microneedle of the present disclosure can be detected by using a chromogenic reaction. A specific example of operation was as follows: 0.15 g of polyvinyl alcohol or a small molecule polyhydroxy compound was taken and respectively placed into 1.8 ml of water, heated and dissolved, and cooled to room temperature, 10 drops of a copper sulfate aqueous solution (17.4% w/w) were added, and the pH value of the solution was adjusted to be 14 or more by a sodium hydroxide aqueous solution (10% w/w). At this time, since a soft green chelate of the polyvinyl alcohol and the divalent cupric ions was insoluble in water, a soft green solid appeared in the polyvinyl alcohol solution. Since a brilliant blue complex of the polyhydroxy compound and the cupric ions was soluble in water, the polyhydroxy compound solution turned to brilliant blue.

After a blank microneedle tablet was crushed, 0.2 g of the crushed tablet was placed into 1.8 ml of water, heated and dissolved, and cooled to room temperature; and 6 drops of a copper sulphate aqueous solution (17.4% w/w) were added and the pH of the solution was adjusted to about 8 by a sodium hydroxide aqueous solution (10% w/w). At this time, a soft green solid appeared, but the solution still kept light greenish blue of the divalent cupric ions or turned substantially colorless. 4 drops of a copper sulfate solution were continuously added into the separated solution, and then a sodium hydroxide solution was added to a pH of 14 or more. At this time, the solution turned brilliant blue. The produced soft green solid was a chelate formed by the polyvinyl alcohol and the cupric ions, and the produced brilliant blue solution was a water-soluble complex formed by the polyhydroxy substance and the divalent cupric ions. 10 drops of a copper sulfate solution may also be added at a time, and then a sodium hydroxide solution was added to a pH of 14 or more. At this time, the soft green solid and the brilliant blue liquid were simultaneously produced. If a layering phenomenon was not obvious, the solution can be centrifugally layered, or 2-4 ml of water was added. The solution was uniformly shaken and stood until the phenomenon was obvious.

The drug-loaded microneedle of the present disclosure can be identified by the same method. If the loaded drug had an influence on the identification, the tip of the drug-loaded microneedle can be cut off first and then the microneedle was identified by the same method.

### Example 11: hydrogen proton nuclear magnetic spectrum detection of microneedle material

The microneedle of the present disclosure can be detected by hydrogen proton nuclear magnetic detection. The specific operations were as follows:
a blank microneedle tablet was crushed, and 1 g of the crushed tablet was placed in 10 ml of water, heated and dissolved, and cooled to room temperature to obtain a solution 1.

30 drops of a 17.4% w/w copper sulfate solution (or a copper chloride solution having the same cupric ion concentration) were added to the other part of solution 1, the pH value was adjusted to 14 or more by a sodium hydroxide aqueous solution (10% w/w), and the solution was centrifugally layered to obtain a solid fraction mainly composed of a polyvinyl alcohol/cupric ion chelate and a liquid fraction mainly composed of a polyhydroxy substance/divalent cupric ion complex respectively.

2 ml of water was added into the solid fraction to form a suspension, and 20% hydrochloric acid was dropwise added into the suspension under shaking till the solid was completely dissolved to form a uniform greenish blue solution; a little zinc powder (200-mesh or more) was added into the solution in several times under shaking until the cupric ions were reduced to a copper element and separated out, and the greenish blue solution became colorless; and the colorless solution was centrifuged and the pH of the solution was adjusted to 7 to obtain a solution 2.

Hydrochloric acid was dropwise added into the liquid fraction until the pH value was 1, and a little zinc powder (200-mesh or more) was added in several times until the solution turned colorless; and the colorless solution was centrifuged and the pH of the solution was adjusted to 7 to obtain a solution 3.

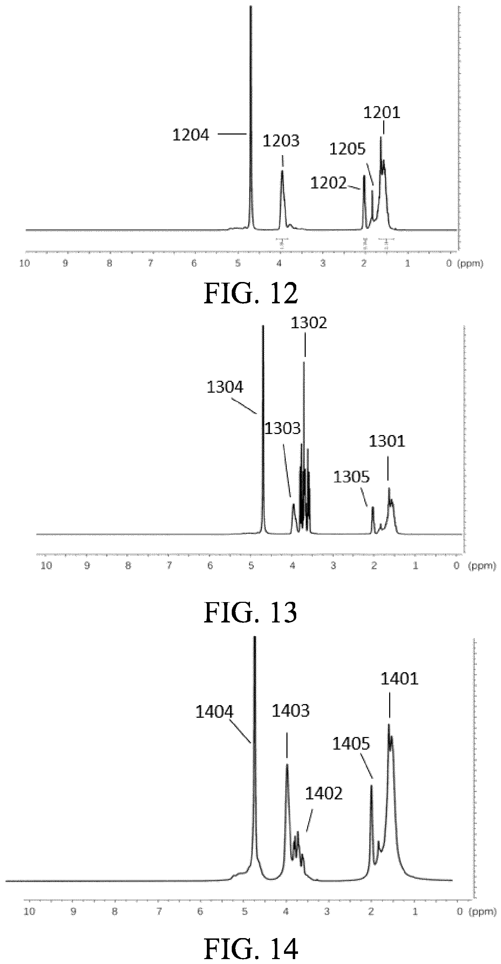

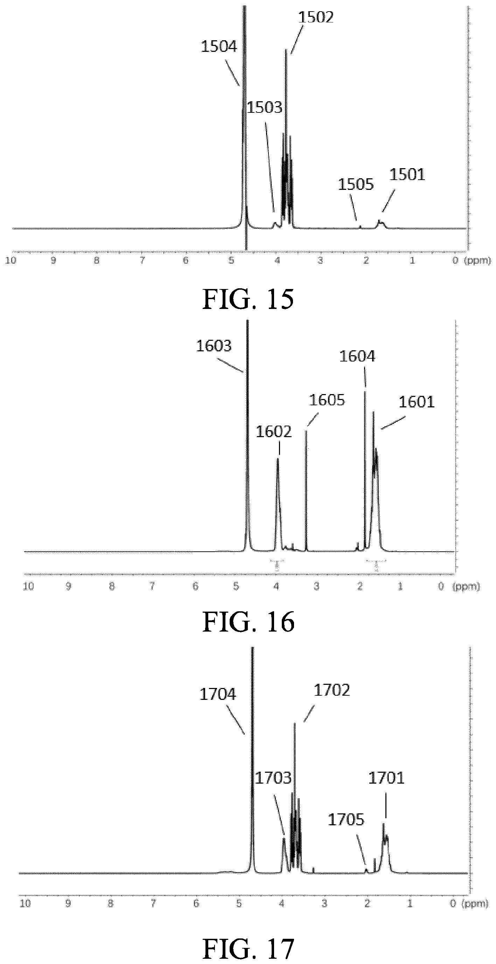

The solution 1, the solution 2, and the solution 3 were respectively dried to obtain a solid 1, a solid 2, and a solid 3; the 3 solids were respectively heated and dissolved with deuterated water (for 100°C/30 minutes or more, the solids were fully shaken to be completely dissolved, and since the polyvinyl alcohol had a high viscosity, the polyvinyl alcohol was slowly dispersed in water); and a nuclear magnetic hydrogen spectrum scanning was conducted on the three deuterated water solutions to obtain a map 1 (FIG. 13 and FIG. 17), a map 2 (FIG. 14 and FIG. 18) and a map 3 (FIG. 15 and FIG. 19).

Comparing with the corresponding hydrogen proton nuclear magnetic spectra of the polyhydroxy substance and the polyvinyl alcohol (FIG. 11, FIG. 12, and FIG. 16), it can be seen that the proton signal of the map 1 was the adduct of the polyvinyl alcohol and the polyhydroxy substance (only manicol was exemplified here, chemical shifts of hydrogen protons of other polyhydroxy substances were very similar to a chemical shift of the hydrogen proton of the manicol, and meanwhile, only 1788 polyvinyl alcohol and 1799 polyvinyl alcohol were exemplified here, and chemical shifts of hydrogen protons of other polyvinyl alcohols were very similar to those of hydrogen protons of 1788 polyvinyl alcohol and 1799 polyvinyl alcohol, such that examples were not provided one by one).

Comparing the map 2 and the map 3 with the map 1 respectively, it can be seen that the proton signal intensity of the polyhydroxy substance in the map 2 was obviously weakened, and the proton signal intensity of the polyvinyl alcohol in the map 3 was obviously weakened.

A chemical shift of -CH2- on the polyvinyl alcohol molecule was δ1.5-1.7 and a chemical shift of -CH- connected with a hydroxyl was about δ4.1. Each -CH2- and -CH- on the polyhydroxy substance were attached to a hydroxy, and the chemical shifts of these protons were in a range of δ3.7-3.9. When factors such as sample concentration, temperature, sample tube rotation speed, and field sweep strength (megahertz number of nuclear magnetic instrument) were comprehensively matched to obtain a better resolution ratio, the -CH- (δ4.1) on the polyvinyl alcohol and the hydrogen proton (δ3.7-3.9) on the polyhydroxy substance can be better distinguished. At this time, through integral calculation of the -CH2- (δ1.5-1.7) on the polyvinyl alcohol molecule and the -CH2- and -CH- (δ3.7-3.9) on the polyhydroxy substance, a quantitative relationship of the approximate matching of the polyvinyl alcohol and the polyhydroxy substance can be obtained.

The hydrogen proton nuclear magnetic spectrum signal was analyzed as follows:
FIG. 11 was a standard H-NMR of manicol, where peak 1101 was -CH3 and -CH2, the solvent was deuterated dimethyl sulfoxide, the position of the peak was slightly more righthand than that in a deuterated water solvent, and peak 1102 was -OH (δ4 or more).

FIG. 12 was an H-NMR of commercially available polyvinyl alcohol 1788, where peak 1201 was -CH2- (δ1.5-δ1.7), peak 1202 was -OCOCH3 (δ2.05), peak 1203 was -CH- (δ4.0-δ4.1) connected with -OH and -OCOCH3, peak 1204 was a water peak, and peak 1205 was an impurity acetate -OCOCH3.

FIG. 13 was an H-NMR of a polyvinyl alcohol 1788/manicol (1:0.6185 w/w) microneedle tablet, where peak 1301 was -CH2- (δ1.5-δ1.7) of the polyvinyl alcohol 1788, peak 1302 was -CH2- and -CH- (δ3.7-3.9) of the manicol, peak 1303 was -CH- (δ4.0-δ4.1) of the polyvinyl alcohol 1788 connected with -OH and -OCOCH3, peak 1304 was a water peak, and peak 1305 was -OCOCH3 (δ2.05) of the polyvinyl alcohol 1788.

FIG. 14 was an H-NMR of a majority of polyvinyl alcohol precipitated by cupric ions of a polyvinyl alcohol 1788/manicol (1:0.6185 w/w) microneedle tablet aqueous solution, where peak 1401 was -CH2- (δ1.5-δ1.7) of the polyvinyl alcohol 1788, peak 1402 was -CH2- and - CH- (δ3.7-3.9) of the manicol, peak 1403 was -CH- (δ4.0-δ4.1) of the polyvinyl alcohol 1788 connected with -OH and -OCOCH3, peak 1404 was a water peak, and peak 1405 was - OCOCH3 (δ2.05) of the polyvinyl alcohol 1788.

FIG. 15 was an H-NMR of a majority of a supernatant manicol after precipitation by cupric ions of a polyvinyl alcohol 1788/manicol (1:0.6185 w/w) microneedle tablet aqueous solution, where peak 1501 was -CH2- (δ1.5-δ1.7) of the polyvinyl alcohol 1788, peak 1502 was -CH2- and -CH- (δ3.7-3.9) of the manicol, peak 1503 was -CH- (δ4.0-δ4.1) of the polyvinyl alcohol 1788 connected with -OH and -OCOCH3, peak 1504 was a water peak, and peak 1505 was - OCOCH3 (δ2.05) of the polyvinyl alcohol 1788.

Comparing FIG. 14 and FIG. 15 with FIG. 13 respectively, it can be seen that the ratio of the polyvinyl alcohol 1788 was significantly increased in a test sample of FIG. 14, and the ratio of the manicol was significantly increased in a test sample of FIG. 15.

FIG. 16 was a self-tested H-NMR of commercially available polyvinyl alcohol 1799, where peak 1601 was -CH2- (δ1.7), peak 1602 was -CH- (δ4.0), peak 1603 was a water peak, peak 1604 was an impurity acetate -OCOCH3, and peak 1605 was an impurity methoxy -OCH3.

FIG. 17 was an H-NMR of a polyvinyl alcohol 1799/manicol (1:0.689 w/w) microneedle tablet, where peak 1701 was -CH2- (δ1.7) of the polyvinyl alcohol 1799, peak 1702 was -CH2- and -CH- (δ3.7-3.9) of the manicol, peak 1703 was -CH- (δ4.0) of the polyvinyl alcohol 1799, peak 1704 was a water peak, and peak 1705 was residual -OCOCH3.

FIG. 18 was an H-NMR of a majority of polyvinyl alcohol precipitated by cupric ions of a polyvinyl alcohol 1799/manicol (1:0.689 w/w) microneedle tablet aqueous solution, where peak 1801 was -CH2- (δ1.7) of the polyvinyl alcohol 1799, peak 1802 was -CH2- and -CH-(δ3.7-3.9) of the manicol, peak 1803 was -CH- (δ4.0) of the polyvinyl alcohol 1799, peak 1804 was a water peak, and peak 1805 was residual -OCOCH3.

FIG. 19 was an H-NMR of a majority of a supernatant manicol after precipitation by cupric ions of a polyvinyl alcohol 1799/manicol (1:0.689 w/w) microneedle tablet aqueous solution, where peak 1901 was an impurity acetate -OCOCH3, peak 1902 was -CH2- and -CH- (δ3.7-3.9) of the manicol, and peak 1903 was a water peak.

Comparing FIG. 18 and FIG. 19 with FIG. 17 respectively, it can be seen that the ratio of the polyvinyl alcohol 1799 was significantly increased in a test sample of FIG. 18, and the ratio of the manicol was significantly increased in a test sample of FIG. 19.

In the above operation, the zinc powder was added to reduce the cupric ions into the copper element, and the copper element was precipitated and removed by separation to avoid the cupric ions from interfering a hydrogen spectrum field sweep; the pH value of the solution was adjusted to about 7 so as to ensure that an acetyl group on the polyvinyl alcohol molecule not completely alcoholyzed was not hydrolyzed to fall off by the catalysis of acid and alkali when the solution was evaporated dry.

The prepared drug-loaded microneedle can be identified by the same method as that of the blank microneedle. If the loaded drug had an influence on the identification, the tip of the drug-loaded microneedle can be cut off first and then the microneedle was identified by the same method.

### Example 12: drug-effect test of insulin microneedle

Model pig of diabetes: three 2-month-old male Bama pigs (average body weight of 8.8 kg) were purchased, fed for three weeks (average body weight of 11 kg), and intravenously injected with streptozotocin (YEASEN Biotechnology (Shanghai) Co., Ltd.) at a dose of 120 mg/kg; and one week later, based on the blood sugar elevation, the pigs were intravenously administrated with the drug at a dose of 50 mg/kg or more as appropriate until fasting blood sugar in the morning was higher than 11 mmol/l, fasting continued for 3 h or more, and the model pig of diabetes was used for a test of the present disclosure.

Test samples: 1. control drug, an insulin injection (Xuzhou Wanbang Biopharmaceuticals, Test Nos. B1 and B2); 2. self-made insulin microneedle for test (Test Nos. A1 to A6): insulin powder (Xuzhou Wanbang Biopharmaceuticals) was placed in a 3% povidone aqueous solution (a weight ratio of the insulin to the aqueous solution was 2:8) to be mixed uniformly; and the solution containing insulin was loaded on a tip of a microneedle made of polyvinyl alcohol 1799/erythritol (1:0.56 w/w); and 3. self-made insulin microneedle for comparison (test No. C1): the same solution containing insulin was loaded on the tip of the self-made microneedle made of an ethylene-vinyl alcohol copolymer; the average drug-loading capacity of the microneedle for test and the microneedle for comparison was 9 units (maximum error +/-9%), so as to prevent the situation that the drug effect observation was influenced by too low dosage.

Test process: each test was conducted every other a normal feeding day. Each test: fasting blood sugar was measured at eight in the morning, the pigs were administered (the control drug was subcutaneously injected, the insulin microneedle was pasted to the ears of the model pigs with one tablet of the self-made insulin microneedle for test each time and two tablets of the self-made insulin microneedle for comparison each time), the blood sugar was measured once per hour, the test was ended after three hours, and the pigs were fed.

The test result was plotted in FIG. 21 and the result of the drug-effect test of the insulin drug-loaded microneedle was shown in the table in FIG. 32.

It can be seen from the table in FIG. 32 that the three-hour blood sugar-reducing mean value of the administration of one tablet of the insulin microneedle loaded by the polyvinyl alcohol 1799/erythritol (1:0.56 w/w) substrate each time on the model pig of diabetes was 6.77 (6 groups of data were obtained by six repeated tests: A1 to A6); the three-hour blood sugar-reducing value of the administration of two tablets of the insulin microneedle loaded by the ethylene-vinyl alcohol copolymer substrate (C1) each time was 5.4, and the mean value of each tablet was only 2.7; and the blood sugar-reducing mean value of 7.5 unit insulin injections (test for twice: B1 to B2) was 8.85.

Namely, the effective drug dose of each tablet of the insulin microneedle loaded by the polyvinyl alcohol 1799/erythritol substrate was 2.5 times that of the insulin microneedle loaded by the ethylene-vinyl alcohol copolymer substrate. It can be presumed that the insulin was quite firmly adsorbed to the surface of the polyvinyl alcohol 1799/erythritol substrate microneedle, and when the tip of the microneedle was pushed into the skin, more drugs were taken into the skin. However, the adsorption force of the surface of the ethylene-vinyl alcohol copolymer substrate microneedle was insufficient, and a part of the drug was pushed out of the skin. In the same way, the drug of the microneedle was softened after entering into the skin to absorb body fluid, and the polyvinyl alcohol 1799/erythritol substrate microneedle still can keep adsorption of the soft drug. However, the ethylene-vinyl alcohol copolymer substrate microneedle had insufficient adsorption force to the soft drug and was possibly extruded outside the skin.

### Example 13: exenatide drug-loaded microneedle and druggability test

Model pig of type II diabetes: eight 2-month-old male Bama pigs (average body weight of 9.1 kg) were purchased, fed for three weeks (average body weight of 12 kg), and intravenously injected with streptozotocin (YEASEN Biotechnology (Shanghai) Co., Ltd.) at a dose of 120 mg/kg; and one week later, based on the blood sugar elevation, the pigs were intravenously administrated with the drug at a dose of 50 mg/kg or more as appropriate until fasting blood sugar in the morning was higher than 11 mmol/l, fasting continued for 3 h or more, and the model pig of diabetes was established. 5 ug of exenatide was injected into the model pig of diabetes, the blood sugar was observed, and one pig with a blood sugar-reducing response to exenatide was selected for test.

Test samples: 1. control drug, an exenatide injection (dose: 5 ug, purchased, and trade name: Byetta); 2. self-made exenatide microneedle for test (24 needles and 120 needles): exenatide powder (Sichuan Shengnuo Technology) was placed into a 3% povidone aqueous solution (a weight ratio of exenatide to povidone aqueous solution was 2:98) to be mixed uniformly; and the solution containing exenatide was loaded on the tip of the microneedle made of polyvinyl alcohol 1799/erythritol (1:0.56 w/w).

Test process: each test was conducted every other a normal feeding day. Each test: fasting blood sugar was measured at eight in the morning, the pigs were administered (the control drug was subcutaneously injected, and the exenatide microneedle was pasted to the ears of the model pigs), the blood sugar was measured once per hour, the test was ended after three hours, and the pigs were fed. The test value of the fasting state without administration was a blank fasting control.

The test result was plotted in FIG. 22 and the result of the drug-effect test of the exenatide drug-loaded microneedle was shown in the table in FIG. 33. The test data indicated:
1. the drug effect of the microneedle with the number of 24 needles was completely the same as that of the microneedle with the number of 120 needles, which indicated that the limitation on the drug-loading capacity was extremely small;
2. the drug effect of the injection decreased in the last two hours because the half-life of the exenatide was short, but the microneedle had a long-acting effect; and
3. the exenatide drug-loaded microneedle had the equivalent drug effect as the injection of 5 ug. When 5 ug of the injection was injected, the blood sugar was reduced faster in the first 1 hour, but the blood sugar-reducing degree was reduced faster in the following 2 hours. In the case of the microneedle, the decrease was slow in the following 2 hours. This indicated that the drug-loaded microneedle had the effect of slowly releasing the drug. Although the number of the 120 needles was greatly different from that of the 24 needles, the drug effect (blood sugar-reducing) degree was comparable, consistent with the reported result that the blood sugar-reducing effect of the exenatide was almost independent of the dose dependence.

### Example 14: insulin/exenatide integrated combined microneedle, and druggability, drug safety, and drug stability thereof

After the secretion equivalent of insulin in a patient with type II diabetes is reduced by 8 IU, the doctor encourages to use insulin very much. After the secretion equivalent is reduced to a certain extent, the doctor encourages to use insulin and exenatide simultaneously very much, but the two injections cannot be mixed together and can be administrated by two injections. Since there are one hundred million patients with type II diabetes, an insulin/exenatide integrated combined microneedle should be a significantly improved new drug with great innovation. As the insulin microneedle and the exenatide microneedle both have been developed successfully and showed a good curative effect, the large semicircular insulin drug-loaded microneedle and the small semicircular exenatide drug-loaded microneedle tablet can be respectively prepared by slightly changing a mold, and then the microneedles were mechanically stuck and fixed on the base plate with the same circle size to form a combined microneedle for applying the two drugs at one time. As the two drugs were respectively loaded on different needle bodies and not in contact with each other, the druggability, the drug safety, and the drug stability of the two drugs are the same as those of the respective monomer drug microneedles as shown in FIG. 20, 20 was a full needle, 201 was a microneedle large semicircle loading one drug, 202 was a microneedle small semicircle loading the other drug, and 203 was a back plate for bonding.

The concept of the combined microneedle administration had universality and can be used for other administration solutions requiring combined medication.

### Example 15: directly pressing blended powder into wafer

Test results of materials, ratio, temperature/pressure, hydrophilicity, and toughness of molded material of wafer directly pressed by blended powder materials

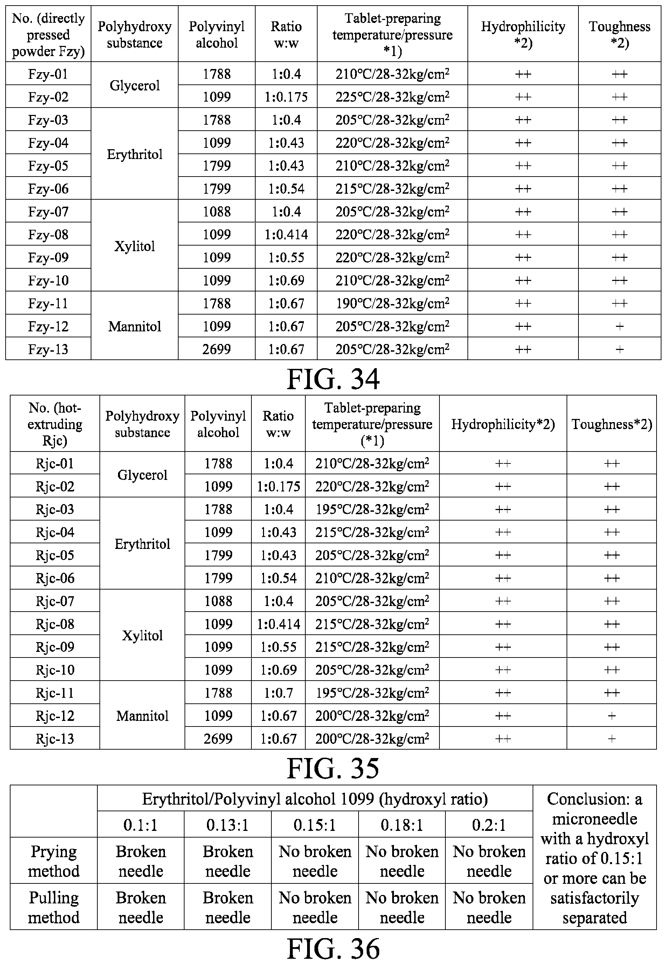

The simple operation process for manufacturing the substrate wafer was as follows: first the device was built according to the sequence shown in FIG. 1, the base plate of the same material replaced the mold plate, the blended powder materials were placed in the through holes of an upper plate, and the pressing device was closed for pressing; and the device was heated to a molding temperature, pressurized and heat-preserved for 27 minutes, and cooled to separate out a wafer. The material selection, ratio, temperature, pressure, surface hydrophilicity, and toughness of the pressed partial wafer were shown in the table in FIG. 34.
Note: 1. the pressure of the used molded green rectangular compressed spring was 28-32 kg/cm²; and
2. + indicated good and ++ indicated very good.

### Example 16: mechanically hot-blending and extruding blended powder into particle material and then preparing into wafer

Test results of materials, ratio, temperature/pressure, hydrophilicity, and toughness of a wafer prepared after mechanically hot-blending and extruding a blended powder into a particle material

The simple operation process for manufacturing the substrate wafer was as follows: firstly the device was built according to the sequence shown in FIG. 1, the base plate of the same material replaced the mold plate, the mechanically hot-blended and extruded particle material was placed in the through holes of an upper plate, and the pressing device was closed for pressing; and the device was heated to a molding temperature, pressurized and heat-preserved for 27 minutes, and cooled to separate out a wafer. The material selection, ratio, temperature, pressure, surface hydrophilicity, and toughness of the pressed partial wafer were shown in the table in FIG. 35.
Note: 1. the pressure of the used molded green rectangular compressed spring was 28-32 kg/cm²; and
2. + indicated good and ++ indicated very good.

### Example 17: separation test of blank microneedle and mold plate

On a mold plate with the thickness of 1.5 mm, materials with hydroxyl ratios of the erythritol to the 1099 polyvinyl alcohol at 0.1:1, 0.13:1, 0.15:1, 0.18:1, and 0.2:1 were respectively used for manufacturing the microneedle; and the thickness of the circular back plate of the microneedle was 1 mm, so as to ensure the pulling stress on the needle body.

Separation method: 1. the mold plate was stabilized and the microneedle was pried by a metal sheet; and 2. the mold plate was stabilized and the back of the microneedle was sucked by a sucker to be vertically pulled. After the separation was finished, the form of the needle body was observed by a 30x magnifying glass. The result of the integrity conditions of the needle bodies when the microneedles with materials of five different ratios were separated from the mold plate was shown in the table in FIG. 36. The microneedle with the materials of the hydroxyl ratio at 0.15:1 or more can be more satisfactorily separated.

The erythritol and polyvinyl alcohol 1099 were selected for the test because the toughness of the material combining the erythritol and the polyvinyl alcohol was ideal. The toughness of different materials with different ratios was different, and the protection scope was not influenced by the difference.

### Example 18: removal test of residual material in pore channel of mold plate

Mannitol was respectively mixed with 1099, 1799, 2499, 2699, 3299, and 3599 polyvinyl alcohols at a weight ratio of 0.65:1 on a mold plate with the thickness of 1.5 mm to obtain microneedles. The wafer of the back layer of the microneedle was cut off, but the needle body was still buried in the pore channel of the mold.

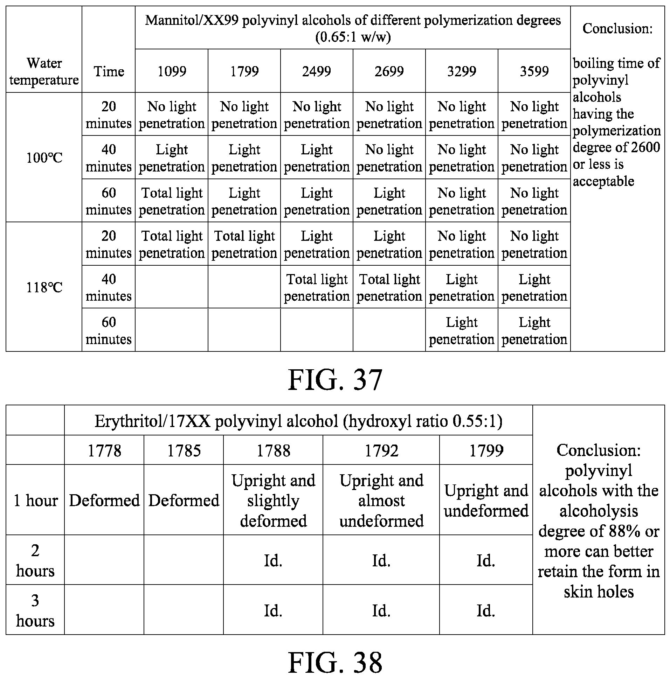

Removal method: 1. stirring with boiling water under a normal pressure (100°C); and 2. stirring with boiling water under pressurization (118°C); the mold plate stood vertically in the water; after the material was hot-dissolved in water for a certain time, the mold was taken out, the water in the hole was volatilized, and the condition of no light penetration (blockage), light penetration (reduced blockage) or total light penetration (removed) of the pore channel was observed by a 30x magnifying glass. The result of the hot-dissolved removal condition of mannitol/XX99 polyvinyl alcohols of different polymerization degrees (0.65:1 w/w) was shown in the table in FIG. 37. The boiling of the polyvinyl alcohols having the polymerization degree of 2600 or less was acceptable.

The mannitol had a relatively low water solubility, the XX99 polyvinyl alcohols had a relatively slow dissolution rate, the mannitol and the XX99 polyvinyl alcohols were selected for strict investigation without influencing the principle concept and protection scope.

### Example 19: test of form retention of 17XX polyvinyl alcohol material needle bodies with different alcoholysis degrees in skin holes

The needle bodies of different materials had different capabilities in retaining forms in the skin holes. In a greenhouse with the temperature of about 37°C and the relative humidity of about 75%, the blank microneedles made of different materials were pasted to arms, the arms were wrapped appropriately, the temperature of the microenvironment was 37°C and the relative humidity was 95% or more by inserting a temperature and humidity probe, and the operation was kept for 1 hour, 2 hours, and 3 hours respectively; and the form of the unloaded microneedles was observed by a 30x magnifying glass, the test result of the form retention of the material needle bodies with different alcoholysis degrees in skin holes was shown in the table in FIG. 38, and the polyvinyl alcohols with the alcoholysis degree of 88% or more can better retain the form in the skin holes.

The polyvinyl alcohols having the same alcoholysis degree and different polymerization degrees had different swelling properties, and the protection scope was not influenced due to examples being provided one by one.

## Claims

1. A strongly hydrophilic microneedle substrate composition, comprising a small molecule polyhydroxy compound and polyvinyl alcohol, wherein a hydroxyl ratio of the small molecule polyhydroxy compound to the polyvinyl alcohol being 0.1:1 to 1.3:1, the polymerization degree of the polyvinyl alcohol being 300-3,500, and the alcoholysis degree being 80% or more.

2. The strongly hydrophilic microneedle substrate composition according to claim 1, wherein the polymerization degree of the polyvinyl alcohol is 300-2,600.

3. The strongly hydrophilic microneedle substrate composition according to claim 1, wherein the alcoholysis degree of the polyvinyl alcohol is 88% or more.

4. The strongly hydrophilic microneedle substrate composition according to claim 1, wherein the hydroxyl ratio of the small molecule polyhydroxy compound to the polyvinyl alcohol is 0.15:1 to 1:1.

5. The strongly hydrophilic microneedle substrate composition according to claim 1, wherein the small molecule polyhydroxy compound is selected from one or more of glycerol, erythritol, threitol, xylitol, arabitol, ribitol, sorbitol, mannitol, galactitol, iditol, volemitol, ribose, xylose, and glucose.

6. The strongly hydrophilic microneedle substrate composition according to claim 1, wherein the small molecule polyhydroxy compound is selected from one or more of glycerol, erythritol, xylitol, sorbitol, and mannitol.

7. A strongly hydrophilic microneedle, a microneedle substrate being the strongly hydrophilic microneedle substrate composition according to any one of claims 1 to 6.

8. A strongly hydrophilic microneedle substrate, the strongly hydrophilic microneedle substrate composition according to any one of claims 1 to 6 being mixed into a blended powder, or the strongly hydrophilic microneedle substrate composition according to any one of claims 1 to 6 being mixed and hot-pressed into a molded material, or the strongly hydrophilic microneedle substrate composition according to any one of claims 1 to 6 being hot-mixed and melt-extruded by a blending machine into a molded material.

9. A method for preparing a strongly hydrophilic microneedle, comprising:
a) blending the strongly hydrophilic microneedle substrate composition according to any one of claims 1 to 6 at room temperature to form a blended powder; and
b) hot-pressing the blended powder into a molded material and hot-pressing the molded material into a mold to form a blank microneedle; or hot-mixing and melt-extruding the blended powder by a blending machine at a certain temperature into a molded material, and hot-pressing the molded material into a mold to form a blank microneedle.

10. The method for preparing a strongly hydrophilic microneedle according to claim 9, wherein the mold is a flat plate with one or more groups of micropore arrays, the micropore is a through hole, and the thickness of the flat plate is 0.2-3 mm; and the molded material is hot-pressed into the mold to form the blank microneedle, namely the blended powder or the molded material is added on the micropore array of the mold plate, and heated to form a melt flow material able to flow, a pressure is applied from an upper part of the material downwards, or vacuumizing is conducted from a lower part of the mold to reduce pressure, or a pressure is applied from the upper part and vacuumizing is conducted from the lower part at the same time so as to enable the melt flow material to enter the micropores of the mold.

11. The method for preparing a strongly hydrophilic microneedle according to claim 10, wherein the pressure is 28-32 kg/cm², and a temperature of the hot-pressing or hot-mixing is from a molding lower limit temperature of the substrate to a decomposition temperature of polyvinyl alcohol.

12. A drug-loaded microneedle, the drug-loaded microneedle being formed by contacting and adsorbing a solution containing a drug on a surface of a tip of the strongly hydrophilic microneedle according to claim 7 and drying the microneedle.

13. The drug-loaded microneedle according to claim 12, wherein the drug is a protein drug, a polypeptide drug or a small molecule drug.

14. The drug-loaded microneedle according to claim 13, wherein the drug is one of insulin, exenatide, human growth hormone, and finasteride.

15. The drug-loaded microneedle according to claim 12, wherein the solution containing a drug is an aqueous solution prepared when the drug is water-soluble, or a solution or suspension prepared by uniformly suspending the drug in water or dissolving/suspending the drug in a non-aqueous polar solvent when the drug is water-insoluble.

16. The drug-loaded microneedle according to claim 12, wherein when the drug is water-soluble, the solution containing a drug consists of a drug, povidone or polyvinyl alcohol, and water, wherein the concentration of the povidone or the polyvinyl alcohol is 0.5%-15%; when the active drug is water-insoluble, the solution containing a drug consists of a drug, povidone or polyvinyl alcohol, and water and/or a non-aqueous polar solvent, wherein the concentration of the povidone or the polyvinyl alcohol is 4%-15%; and the non-aqueous polar solvent is one or a mixed solvent of methanol, ethanol, and isopropanol.

17. The drug-loaded microneedle according to claim 12, wherein the contacting and adsorbing process comprises: forming a groove with the depth of 0.1-0.7 mm in a flat plate, adding the solution containing a drug into the groove to a certain height, enabling a tip of the strongly hydrophilic microneedle to vertically face downwards, enabling the microneedle to stand and stay in the circular groove, taking out the microneedle, and drying the microneedle to obtain the drug-loaded microneedle.

18. A drug-loaded microneedle for combined administration, formed by integrally combining two or more microneedles each loading a different drug of the drug-loaded microneedle according to any one of claims 12 to 14.

19. The combined drug-loaded microneedle according to claim 18, formed by integrally combining an insulin drug-loaded microneedle and an exenatide drug-loaded microneedle, wherein the insulin drug-loaded microneedle and the exenatide drug-loaded microneedle are separately arranged.

20. An insulin drug-loaded microneedle, wherein in the drug-loaded microneedle according to any one of claims 12 to 15, the drug is insulin.

21. An exenatide drug-loaded microneedle, wherein in the drug-loaded microneedle according to any one of claims 12 to 15, the drug is exenatide.
